# EUROPEAN PATENT SPECIFICATION

(11) **EP 3 131 535 B1**
(45) Date of publication and mention of the grant of the patent: **29.04.2020**
(21) Application number: 15779784.6
(22) Date of filing: 26.03.2015
(51) Int. Cl.: A61K 9/56, A61K 31/205, A61K 31/606

(54) **PROCESS FOR PREPARATION OF MESALAMINE COMPOSITION AND MESALAMINE COMPOSITION THEREOF**
VERFAHREN ZUR HERSTELLUNG EINER MESALAMINZUSAMMENSETZUNG SOWIE MESALAMINZUSAMMENSETZUNG DAVON
PROCÉDÉ DE PRÉPARATION D'UNE COMPOSITION DE MÉSALAMINE ET COMPOSITION DE MÉSALAMINE AINSI OBTENUE

(30) Priority: 17.04.2014 IN 1393MU2014
(43) Date of publication of application: 22.02.2017
(73) Proprietor: Athena Drug Delivery Solutions Pvt Ltd., Andheri (East) Mumbai 400059 (IN)
(72) Inventor: B. CHAUDHARI, Mahendra, Thane (West) Maharashtra 400602 (IN); S. WADHWANI, Jagdish, Ulhasnagar (East) Maharashtra 421005 (IN); P. NEHETE, Nitin, Dombivili (E) Maharashtra 421201 (IN)
(74) Representative: Papula Oy
(86) International application number: PCT/IN2015/000143
(87) International publication number: WO 2015/159302

(56) References cited:
- WO-A1-2006/069030
- WO-A1-2011/015964
- WO-A2-2006/130703
- WO-A2-2012/127495
- US-A- 5 885 616
- US-A1- 2007 043 004
- SINGH DEEP HUSSAN ET AL: "A review on recent advances of enteric coating", IOSR JOURNAL OF PHARMACY, INTERNATIONAL ORGANIZATION OF SCIENTIFIC RESEARCH, IN, vol. 2, no. 6, 1 November 2012 (2012-11-01), pages 5-11, ISSN: 2319-4219, DOI: 10.9790/3013-2610511

## Description

### FIELD OF THE INVENTION

The present invention relates to the process for preparing mesalamine composition; and composition of the same.

### BACKGROUND OF THE INVENTION

Mesalamine is used to treat ulcerative colitis (a condition in which part or all of the lining of the colon [large intestine] is swollen or worn away). mesalamine delayed-release tablets and controlled-release capsules may be used to treat ulcerative colitis that affects any part of the colon. Conventional mesalamine pellets have been available for a long time; however the process for manufacturing of these conventional pellets is not efficiently reproducible and requires a number of machines for conducting the different manufacturing steps which results in a slower process. Further, the appearance of the conventional mesalamine pellets seems very dull and uninteresting. Also, the layering process to prepare the conventional mesalamine pellets does not provide for a high drug content suspension. WO 2011/015964 A1 discloses controlled release granular compositions of mesalazine which comprise a central core comprising an inert substrate, an intermediate layer comprising mesalazine and one or more physiologically acceptable excipients and a gastro-resistant coating.

WO 2006/130703 A discloses a modified release mesalamine oral dosage comprising cores comprising mesalamine, coated with a mixture of water-impermeable polymer and water-swellable polymer.

Accordingly, there is a need for a process to manufacture mesalamine pellets that is faster, simple, robust and reproducible. Further, what is required is a process for manufacturing a mesalamine composition that possesses a high drug content suspension for layering processes and resulting in pellets having an aesthetic appearance.

### SUMMARY OF THE INVENTION

To achieve the foregoing and other objects and needs, the present invention provides a process for preparation of mesalamine composition and mesalamine composition thereof that provides, faster, easier, robust, automated, and reproducible process for preparing mesalamine composition. Also, the present invention provides a process for preparing mesalamine composition wherein all the steps of preparing the mesalamine composition can be performed in a single machine, thereby conducting the process of preparation with lesser number of equipments.

In one aspect, the present invention provides a process for preparation of a mesalamine composition comprising: about 55 to about 65 percent by weight of a mesalamine; about 7 to about 9 percent by weight of a sugar spheres ; about 17 to about 19 percent by weight of a binder wherein the binder is hypromellose; about 0.50 to about 0.80 percent by weight of a surfactant wherein the surfactant is polysorbate 80; about 1 to about 1.5 percent by weight of an anti foaming agent; about 4 to about 5 percent by weight of a release controlling agent wherein the release controlling agent is ethyl cellulose; about 2 to about 3 percent by weight of a pore forming agent wherein the pore forming agent is polyvinyl pyrrolidone; about 0.5 to about 1 percent by weight of a plasticizer wherein the plasticizer is dibutyl sebacate; about 1.5 to about 3 percent by weight of a lubricant wherein the lubricant is purified talc; and about 2.5 to about 3 percent by weight of a colorant. The process comprises: preparing a first drug layering suspension comprising mesalamine; performing a first drug layering in the fluid bed coater by applying the first drug layering suspension on sugar spheres to form first drug layered pellets; preparing a seal coating suspension; performing the seal coating in the fluid bed coater by applying the seal coating suspension on the first drug layered pellets to form seal coated pellets; preparing a second drug layering suspension comprising mesalamine; performing the second drug layering in the fluid bed coater by applying the second drug layering suspension on seal coated pellets to form second drug layered pellets; preparing an extended release coating suspension; performing the extended release coating in the fluid bed coater by applying extended release coating suspension on the second drug layered pellets to form extended release pellets; preparing a color coating suspension; performing the color coating in the fluid bed coater by applying the color coating suspension on the extended release pellets to form color coated pellets; and lubricating the color coated pellets to form mesalamine pellets.

In another aspect, the present invention provides mesalamine composition comprising: about 55 to about 65 percent by weight of a mesalamine; about 7 to about 9 percent by weight of a sugar spheres ; about 17 to about 19 percent by weight of a binder wherein the binder is hypromellose; about 0.50 to about 0.80 percent by weight of a surfactant wherein the surfactant is polysorbate 80; about 1 to about 1.5 percent by weight of an anti foaming agent; about 4 to about 5 percent by weight of a release controlling agent wherein the release controlling agent is ethyl cellulose; about 2 to about 3 percent by weight of a pore forming agent wherein the pore forming agent is polyvinyl pyrrolidone; about 0.5 to about 1 percent by weight of a plasticizer wherein the plasticizer is dibutyl sebacate; about 1.5 to about 3 percent by weight of a lubricant wherein the lubricant is purified talc; and about 2.5 to about 3 percent by weight of a colorant.

### BRIEF DESCRIPTION OF THE DRAWINGS

The advantages and features of the present invention will become better understood with reference to the following detailed description and claims taken in conjunction with the accompanying drawing, in which:
FIG. 1 illustrates a process flow for preparation of a mesalamine composition, in accordance with an exemplary embodiment of the present invention;
FIG. 2 illustrates details of preparation of first drug layering suspension of process of FIG. 1;
FIG. 3 illustrates details of preparation of first drug layered pellets of process of FIG. 1;
FIG. 4 illustrates details of preparation of seal coating suspension of process of FIG. 1;
FIG. 5 illustrates details of preparation of seal coated pellets of process of FIG. 1;
FIG. 6 illustrates details of preparation of second drug layering suspension of process of FIG. 1;
FIG. 7 illustrates details of preparation of second drug layered pellets of process of FIG. 1;
FIG. 8 illustrates details of preparation of extended release coating suspension of process of FIG. 1;
FIG. 9 illustrates details of preparation of extended release pellets of process of FIG. 1;
FIG. 10 illustrates details of preparation of color coating suspension of process of FIG. 1;
FIG. 11 illustrates details of preparation of color coated pellets of process of FIG. 1; and
FIG. 12 illustrates details of preparation of mesalamine pellets of process of FIG. 1.

### DETAILED DESCRIPTION OF THE INVENTION

The use of terms "including," "comprising," or "having" and variations thereof herein is meant to encompass the items listed thereafter as well as additional items. Further, the terms, "a" and "an" herein do not denote a limitation of quantity, but rather denote the presence of at least one of the referenced item.

The present invention provides a process for preparation of mesalamine composition (hereinafter referred to as process); and mesalamine composition thereof. The process comprises performing all the steps of drug layering, seal coating, extended release coating, and color coating in a single automated fluid bed coater. Specifically, all the steps of preparing the mesalamine composition can be performed in a single machine, thereby conducting the process of preparation with lesser number of equipments. Accordingly, the present invention provides a faster, easier, robust, automated and reproducible process for preparation of a mesalamine composition.

The process also provides for a drug layering in a fluid bed coater for mesalamine compositions.

The pellets of the mesalamine composition formed by the process are spherical in shape and have excellent uniformity. Further the process employs a color coating to provide aesthetic value to the mesalamine composition and enhances the appearance of the mesalamine composition formed by the process. Also, the process provides for the preparation of a mesalamine composition having an intermediate rate controlling seal coating for better release control purpose, and extended release coating to enhance patient compliances and for reducing the dosage intervals.

As used herein, the mesalamine also known as mesalazine or 5-aminosalicylic acid is an anti-inflammatory drug used to treat inflammatory bowel disease, such as ulcerative colitis and mild-to-moderate crohn's disease. The mesalamine is a bowel-specific aminosalicylate drug that acts locally in the gut and has its predominant actions there, thereby having few systemic side effects. As used in the present invention, mesalamine is obtained from Sun pharmaceutical. Further as used herein mesalamine pellets are the small particles created by compressing the processed mesalamine formed by the process as described below.

The process of the present invention is defined in claim 1, and comprises the steps of: a first drug layering; a seal coating; a second drug layering; an extended release coating; a color coating; lubrication and optionally packing.

The mesalamine composition of the present invention comprises: about 55 to about 65 percent by weight of a mesalamine; about 7 to about 9 percent by weight of a sugar spheres; about 17 to about 19 percent by weight of a binder wherein the binder is hypromellose; about 0.50 to about 0.80 percent by weight of a surfactant wherein the surfactant is polysorbate 80; about 1 to about 1.5 percent by weight of an anti foaming agent; about 4 to about 5 percent by weight of a release controlling agent wherein the release controlling agent is ethyl cellulose; about 2 to about 3 percent by weight of a pore forming agent wherein the pore forming agent is polyvinyl pyrrolidone; about 0.5 to about 1 percent by weight of a plasticizer wherein the plasticizer is dibutyl sebacate; about 1.5 to about 3 percent by weight of a lubricant wherein the lubricant is purified talc; and about 2.5 to about 3 percent by weight of a colorant.

As used herein, sugar spheres are of the size about 300µm to about 500µm. Further, as used in the present invention, sugar spheres may be obtained from Salus/ Casteli.

In one embodiment, the binder is methocel E3 (hypromellose). Methocel E3 is a semisynthetic, inert, viscoelastic polymer used as an ophthalmic lubricant, as well as an excipient and controlled-delivery component in oral medicaments, found in a variety of commercial products. Further, as a food additive, methocel E3 is an emulsifier, thickening and suspending agent, and is an alternative to animal gelatin. As used in the present invention, methocel E3 is brand name of Colorcon (Dow) having viscosity 3 CPS and is obtained from Colorcon.

The surfactant is polysorbate 80. Polysorbate 80 is a nonionic surfactant and emulsifier derived from polyethoxylated sorbitan and oleic acid, and is often used in foods. The hydrophilic groups in this compound are polyethers also known as polyoxyethylene groups which are polymers of ethylene oxide. In the nomenclature of polysorbates, the numeric designation following polysorbate refers to the lipophilic group. As used in the present invention, polysorbate 80 may be obtained from Merck.

As used herein, the anti foaming agent is simethicone 30% emulsion. Simethicone is an orally administered anti-foaming agent used to reduce bloating, discomfort or pain caused by excessive gas mainly swallowed air, with small amounts of hydrogen and methane in the stomach or intestines. As used in the present invention, simethicone 30% emulsion may be obtained from Rio-care.

In one embodiment, the release controlling agent is ethocel N 20 (Ethyl cellulose N 20). Ethocel N 20 is a polymer which is inert, high purity powder with no caloric value and is virtually colorless, odorless, and tasteless. They are derived from and have the polymeric "backbone" of cellulose, a naturally occurring polymer. Ethocel N 20 is effective as controlled-release coatings, microencapsulation, granulation, and taste masking. As used in the present invention, ethocel N 20 may be obtained from Colorcon.

In one embodiment, the pore forming agent is Povidone K-30. Povidone K-30 is also commonly known as (PVP K-30). Povidone (polyvinylpyrrolidone, PVP) is used in the pharmaceutical industry as a synthetic polymer vehicle for dispersing and suspending drugs. It has multiple uses, including as a binder for tablets and capsules, a film former for ophthalmic solutions, to aid in flavoring liquids and chewable tablets, and as an adhesive for transdermal systems. The PVP K-30 has the molecular formula of (C₆H₉NO)ₙ and appears as a white to slightly off-white powder. PVP K-30 formulations are widely used in the pharmaceutical industry due to their ability to dissolve in both water and oil solvents. As used in the present invention, Povidone K-30 may be obtained from BASF.

The plasticizer is dibutyl sebacate (DBS). Dibutyl sebacate is an organic chemical, a dibutyl ester of sebacic acid. The dibutyl sebacate main use is as a plasticizer in production of plastics, namely cellulose acetate butyrate, cellulose acetate propionate, ethyl cellulose, polyvinyl butyral, polyvinyl chloride, polystyrene, and many synthetic rubbers (especially nitrile rubber and neoprene) and other plastics. Dibutyl sebacate can be used for plastics in use in the food packaging industry, in plastics used for medical devices, and for pharmaceutical applications, e.g. as a plasticizer for film coating of tablets, beads, and granules. The dibutyl sebacate provides excellent compatibility with a range of plastic materials, superior properties at low temperatures, and good oil resistivity. As used in the present invention, dibutyl sebacate is obtained from Vertellus.

The lubricant is purified talc. The lubricants prevent ingredients from clumping together and from sticking to the tablet punches or capsule filling machine. Further, in pellets formulation, lubricant reduces sticking of the pellets. Lubricants also ensure that tablet formation and ejection can occur with low friction between the solid and die wall. Common minerals like talc or silica, and fats, e.g. vegetable stearin, magnesium stearate or stearic acid are the most frequently used lubricants in tablets or hard gelatin capsules. The lubricants are agents added in small quantities to tablet and capsule formulations to improve certain processing characteristics. As used in the present invention, purified talc may be obtained from Luzenac.

In one embodiment, the colorant is opadry yellow color (colorcon yellow). The colorants or coloring agents which are mainly used to impart a distinctive appearance to the pharmaceutical dosage forms. As used in the present invention, opadry yellow color may be obtained from Ideal cure/ Colorcon.

The process for preparation of the mesalamine composition comprises: preparing a first drug layering suspension comprising mesalamine; performing a first drug layering in the fluid bed coater by applying the first drug layering suspension on sugar spheres to form first drug layered pellets; preparing a seal coating suspension; performing the seal coating in the fluid bed coater by applying the seal coating suspension on the first drug layered pellets to form seal coated pellets; preparing a second drug layering suspension comprising mesalamine; performing the second drug layering in the fluid bed coater by applying the second drug layering suspension on seal coated pellets to form second drug layered pellets; preparing an extended release coating suspension; performing the extended release coating in the fluid bed coater by applying extended release coating suspension on the second drug layered pellets to form extended release pellets; preparing a color coating suspension; performing the color coating in the fluid bed coater by applying the color coating suspension on the extended release pellets to form color coated pellets; and lubricating the color coated pellets to form mesalamine pellets.

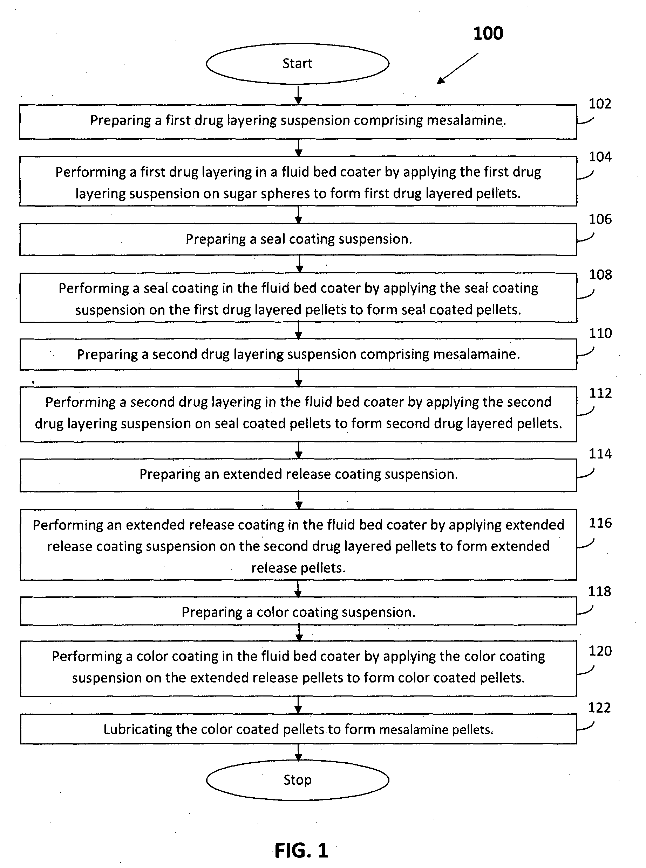

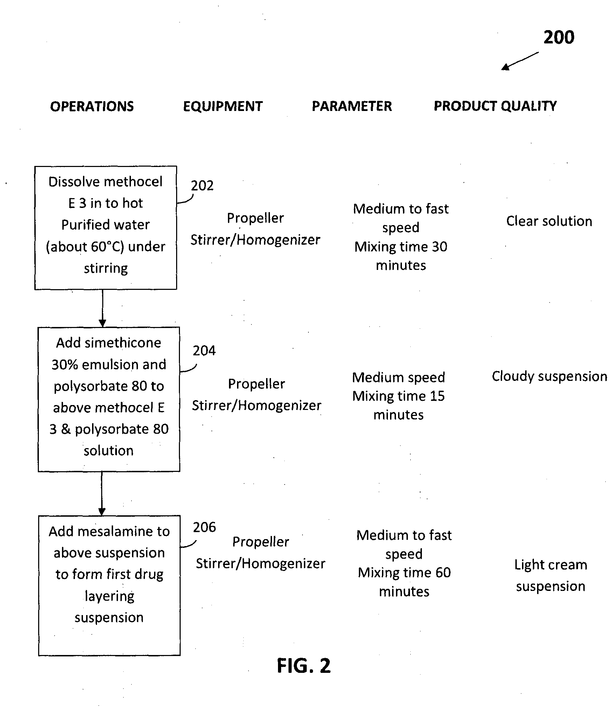

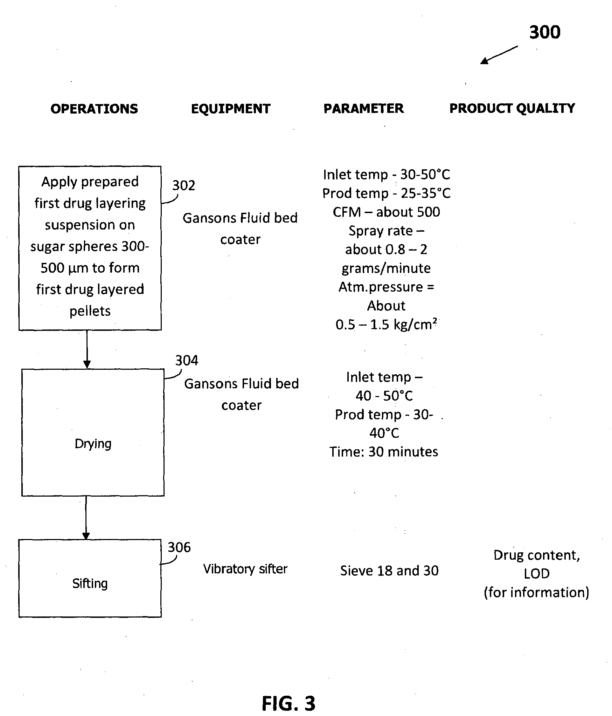

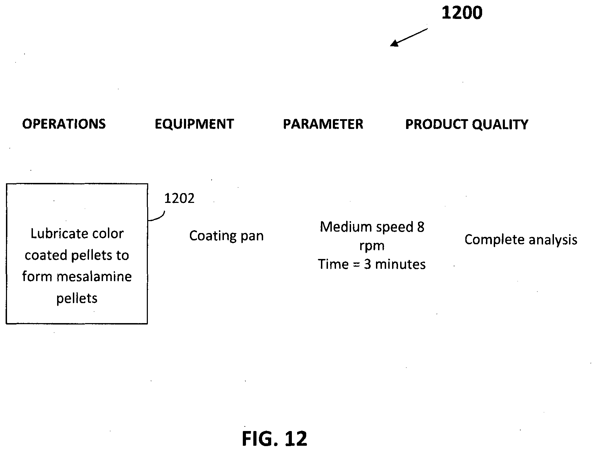

Specifically, the process 100 is described herein with reference to FIGS. 1-12. In FIG. 1, the different process steps are illustrated, while in FIGS. 2-12 are the details of steps illustrated in FIG. 1. Specifically, FIG. 2 and FIG. 3 illustrate details of the first drug layering of process of FIG. 1; FIG. 4 and FIG. 5 illustrate details of seal coating of process of FIG. 1; FIG. 6 and FIG. 7 illustrate details of second drug layering of process of FIG. 1; FIG. 8 and FIG. 9 illustrate details of extended release coating of process of FIG. 1; FIG. 10 and FIG. 11 illustrate details of color coating of process of FIG. 1; and FIG. 12 illustrates details of lubrication of process of FIG. 1. In the below description of the process, the reference numerals are used individually and/or collectively from one or more of the FIGS. 1-12.

Referring to FIG. 1, at step 102, the process 100 initiates by preparing the first drug layering suspension comprising mesalamine. Referring to 202, 204 and 206 of FIG. 2, the first drug layering suspension is formed by mixing hypromellose (methocel E3), hot purified water, simethicone 30% emulsion, polysorbate 80 and mesalamine. Specifically at 202, the methocel E3 is dissolved in to the hot purified water (about 60°C water temperature) under stirring/homogenizing conditions in a propeller stirrer/homogenizer from medium to fast speed for about 30 minutes or till clear solution is formed. At 204, simethicone 30% emulsion and polysorbate 80 is added in to above methocel E3 solution under stirring/homogenizing conditions in propeller stirrer/homogenizer from medium to fast speed for about 15 minutes and cooled to reach room temperature to form cloudy suspension. At 206, mesalamine active pharmaceutical ingredient (API) is added in to above suspension resulted at 204 under stirring/homogenizing conditions in propeller stirrer/homogenizer from medium to fast speed for about 60 minutes or till light cream color suspension is formed. This light cream suspension formed at 206 is the first drug layering suspension.

Now referring again to FIG. 1, at step 104, the process 100 comprises performing a first drug layering in a fluid bed coater (for example, a Gansons fluid bed coater) by applying the first drug layering suspension on sugar spheres to form first drug layered pellets. Referring to 302, 304 and 306 of FIG. 3, the first drug layered pellets is formed by applying the first drug layering suspension formed at 206 of FIG. 2 on sugar spheres. Specifically, at 302, the first drug layering suspension formed at 206 of FIG. 2 is applied on sugar sphere of the size about 300µm to about 500µm in fluid bed coater at pre-determined parameters to form first drug layered pellets. As used herein, pre-determined parameters include an inlet temperature of about 30°C to about 50°C, product temperature of about 25°C to about 35°C, fluidization of air volume of about 500 CFM, spraying rate of the first drug layering suspension of about 0.8 grams to about 2 grams per minute and atmospheric pressure of about 0.5 kg/cm² to about 1.5 kg/cm². Although particular pre-determined parameters are mentioned above, it will be evident to a person skilled in the art to modify predetermined parameters based on obvious variations.

At 304, the first drug layered pellets formed at 302 is dried in the fluid bed coater for 30 minutes at the pre-determined parameters. As used herein, pre-determined parameters include an inlet temperature of about 40°C to about 50°C, and product temperature of about 30°C to about 40°C. Although particular pre-determined parameters are mentioned above, it will be evident to a person skilled in the art to modify predetermined parameters based on obvious variations. The dried first drug layered pellets may have loss on drying (LOD) of not more than 2 percent.

At 306, the dried first drug layered pellets are sifted/sieved in the vibratory sifter of about ASTM 18 to about ASTM 30 to form the dried and sieved first drug layered pellets.

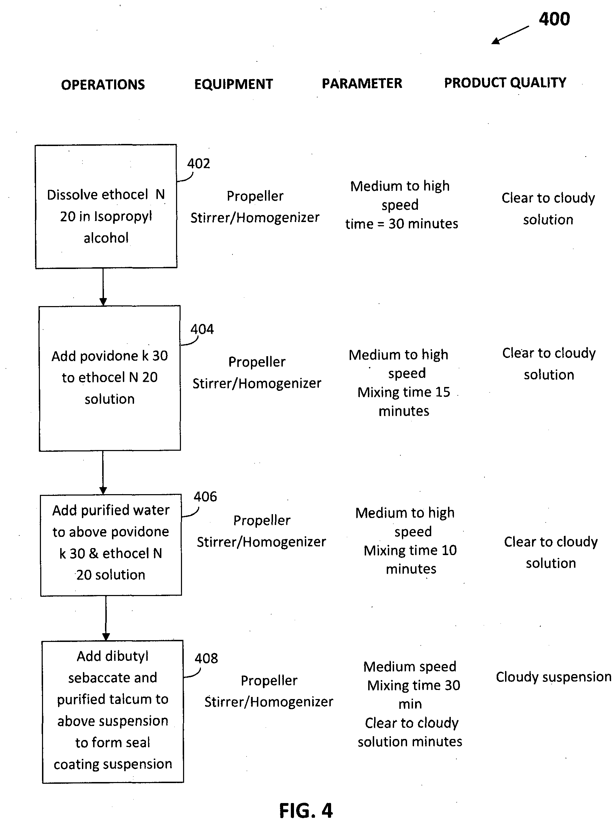

Now referring again to FIG. 1, at step 106, the process 100 comprises preparing a seal coating suspension. Referring to 402, 404, 406 and 408 of FIG. 4, the seal coating suspension is formed by mixing ethocel N 20, isopropyl alcohol, povidone K 30, purified water, dibutyl sebacate and purified talcum. Specifically at 402, ethocel N 20 is dissolved in isopropyl alcohol under stirring/homogenizing conditions in propeller stirrer/homogenizer from medium to high speed for about 30 minutes for a clear to cloudy solution is formed. At 404, povidone K 30 is added in to above ethocel N 20 solution formed at 402 under stirring/homogenizing conditions in propeller stirrer/homogenizer from medium to high speed for about 15 minutes or till clear to cloudy suspension is formed. At 406, purified water is added in to above solution formed at 404 under stirring/ homogenizing conditions in propeller stirrer/homogenizer from medium to high speed for about 10 minutes or till clear to cloudy suspension is formed. At 408, dibutyl sebacate and purified talc is added in to above solution formed at 406 under stirring/ homogenizing conditions in propeller stirrer/homogenizer from medium to high speed for about 30 minutes or till clear to cloudy suspension is formed. This suspension formed at 408 is the seal coating suspension.

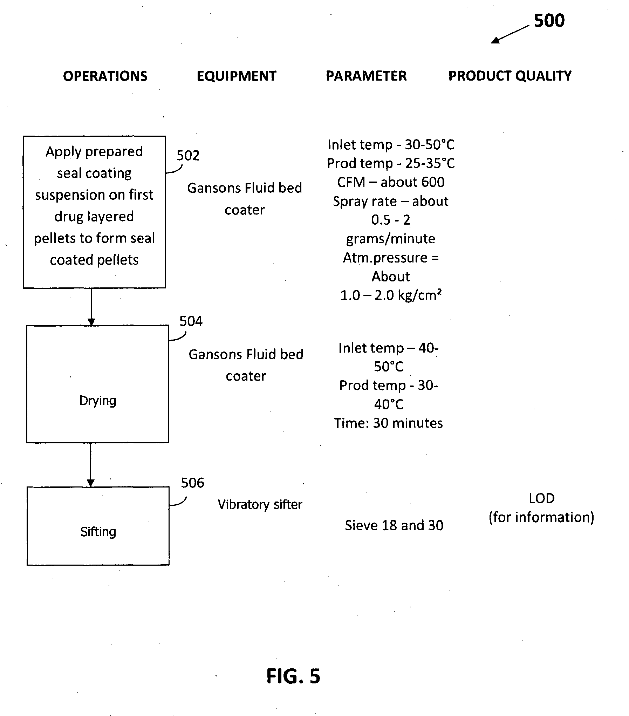

Now referring again to FIG. 1, at step 108, the process 100 comprises performing a seal coating in the fluid bed coater by applying the seal coating suspension on the first drug layered pellets to form seal coated pellets. Referring to 502, 504 and 506 of FIG. 5, the seal coated pellets is formed by applying the seal coating suspension formed at 408 of FIG. 4 on the first drug layered pellets formed at 306 of FIG. 3 to form seal coated pellets. Specifically, at 502, the seal coating suspension formed at 408 of FIG. 4 is applied on the first drug layered pellets formed at 306 of FIG. 3 in fluid bed coater at predetermined parameters to form seal coated pellets. As used herein, predetermined parameters include an inlet temperature of about 30°C to about 50°C, product temperature of about 25°C to about 35°C, fluidization of air volume of about 600 CFM, spraying rate of the seal coating suspension of about 0.5 grams to about 2 grams per minute and atmospheric pressure of about 1.0 kg/cm² to about 2.0 kg/cm². Although particular predetermined parameters are mentioned above, it will be evident to a person skilled in the art to modify predetermined parameters based on obvious variations.

At 504, the seal coated pellets formed at 502 is dried in the fluid bed coater for 30 minutes at predetermined parameters. As used herein, predetermined parameters include an inlet temperature of about 40°C to about 50°C, product temperature of about 30°C to about 40°C. Although particular predetermined parameters are mentioned above, it will be evident to a person skilled in the art to modify predetermined parameters based on obvious variations. The dried first drug layered pellets may have loss on drying (LOD) of not more than 2 percent.

At 506, the dried seal coated pellets are sifted/sieved in the vibratory sifter of about ASTM 18 to about ASTM 30 to form the dried and sieved seal coated pellets.

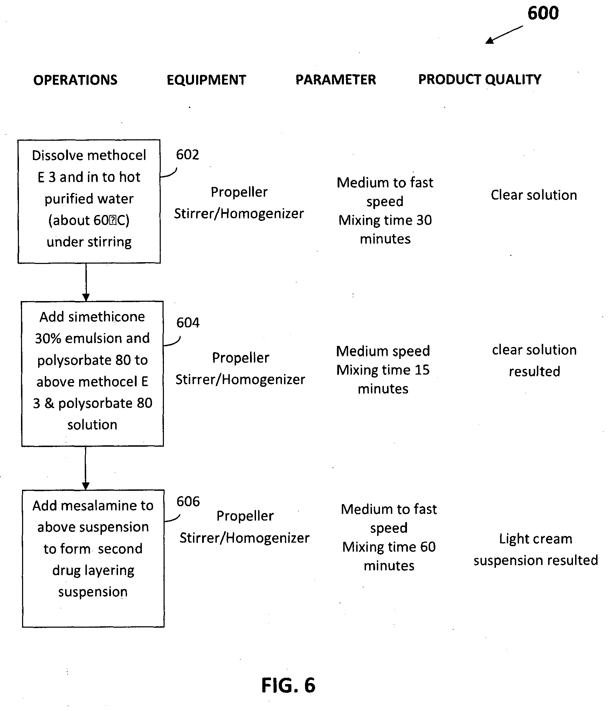

Now referring again to FIG. 1, at step 110, the process 100 comprises preparing a second drug layering suspension comprising mesalamine. Referring to 602, 604 and 606 of FIG. 6, the second drug layering suspension is formed by mixing methocel E3, hot purified water, simethicone 30% emulsion, polysorbate 80 and mesalamine. Specifically at 602, the methocel E3 is dissolved in to the hot purified water (about 60°C) under stirring/ homogenizing conditions in a propeller stirrer/homogenizer from medium to fast speed for about 30 minutes or till clear solution is formed. At 604, simethicone 30% emulsion and polysorbate 80 is added in to above methocel E3 solution under stirring/ homogenizing conditions in a propeller stirrer/homogenizer from medium to fast speed for about 15 minutes and cooled to reach room temperature to form cloudy suspension. At 606, mesalamine is added in to above suspension resulted at 604 under stirring/ homogenizing conditions in a propeller stirrer/homogenizer from medium to fast speed for about 60 minutes or till light cream color suspension is formed. This light cream suspension formed at 606 is the second drug layering suspension.

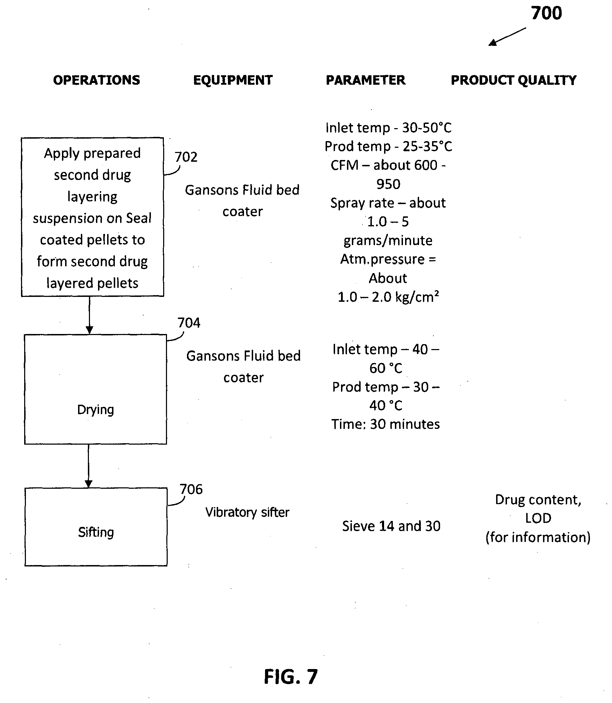

Now referring again to FIG. 1, at step 112, the process 100 comprises performing a second drug layering in the fluid bed coater by applying the second drug layering suspension on seal coated pellets to form second drug layered pellets. Referring to 702, 704 and 706 of FIG. 7, the second drug layered pellets is formed by applying the second drug layering suspension formed at 606 of FIG. 6 on seal coated pellets formed at 506 of FIG. 5. Specifically, at 702, the second drug layering suspension formed at 606 of FIG. 6 is applied on seal coated pellets formed at 506 of FIG. 5 in fluid bed coater at predetermined parameters to form second drug layered pellets. As used herein, predetermined parameters include an inlet temperature of about 30°C to about 50°C, product temperature of about 25°C to about 35°C, fluidization of air volume of about 600 CFM to about 950 CFM, spraying rate of the first drug layering suspension of about 1.0 grams to about 5 grams per minute and atmospheric pressure of about 1.0 kg/cm² to about 2.0 kg/cm². Although particular predetermined parameters are mentioned above, it will be evident to a person skilled in the art to modify the predetermined parameters based on obvious variations.

At 704, the second drug layered pellets formed at 702 is dried in the fluid bed coater for 30 minutes at predetermined parameters. As used herein, predetermined parameters include an inlet temperature of about 40°C to about 60°C, product temperature of about 30°C to about 40°C. Although particular predetermined parameters are mentioned above, it will be evident to a person skilled in the art to modify predetermined parameters based on obvious variations. The dried second drug layered pellets may have loss on drying (LOD) of not more than 2 percent.

At 706, the dried first drug layered pellets are sifted/sieved in the vibratory sifter of about ASTM 14 to about ASTM 30 to form the dried and sieved second drug layered pellets.

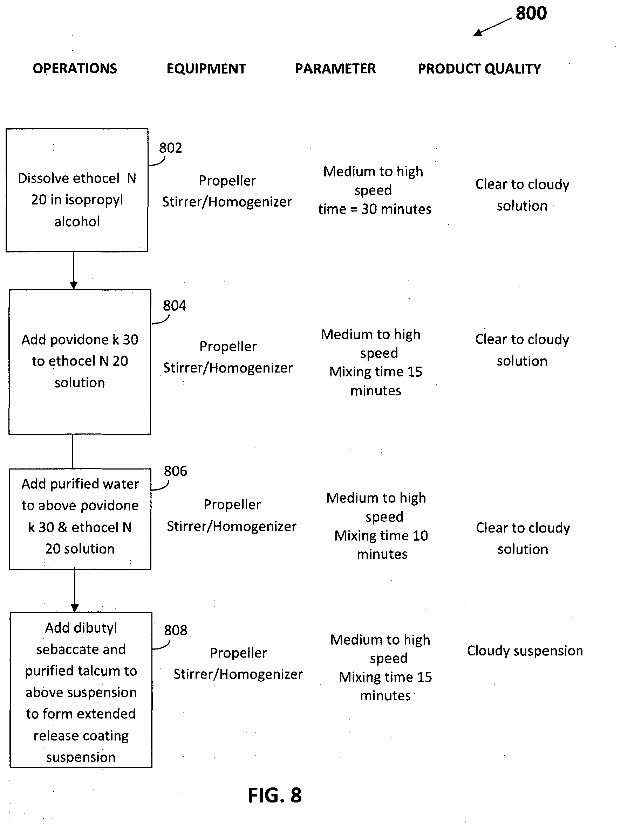

Now referring again to FIG. 1, at step 114, the process 100 comprises preparing an extended release coating suspension. Referring to 802, 804, 806 and 808 of FIG. 8, the extended release coating suspension is formed by mixing ethocel N 20, isopropyl alcohol, povidone K 30, purified water, dibutyl sebacate and purified talcum. Specifically at 802, the ethocel N 20 is dissolved in to the Isopropyl alcohol under stirring/ homogenizing conditions in a propeller stirrer/homogenizer from medium to high speed for about 30 minutes or till cloudy suspension is formed. At 804, povidone K 30 is added in to above ethocel N 20 solution formed at 802 under stirring/ homogenizing conditions in a propeller stirrer/homogenizer from medium to high speed for about 15 minutes or till clear to cloudy suspension is formed. At 806, purified water is added in to above solution formed at 804 under stirring/ homogenizing conditions in a propeller stirrer/homogenizer from medium to high speed for about 10 minutes or till clear to cloudy suspension is formed. At 808 dibutyl sebacate and purified talc is added in to above solution formed at 806 under stirring/ homogenizing conditions in a propeller stirrer/homogenizer from medium to high speed for about 15 minutes or till clear to cloudy suspension is formed. This cloudy suspension formed at 808 is the extended release coating suspension.

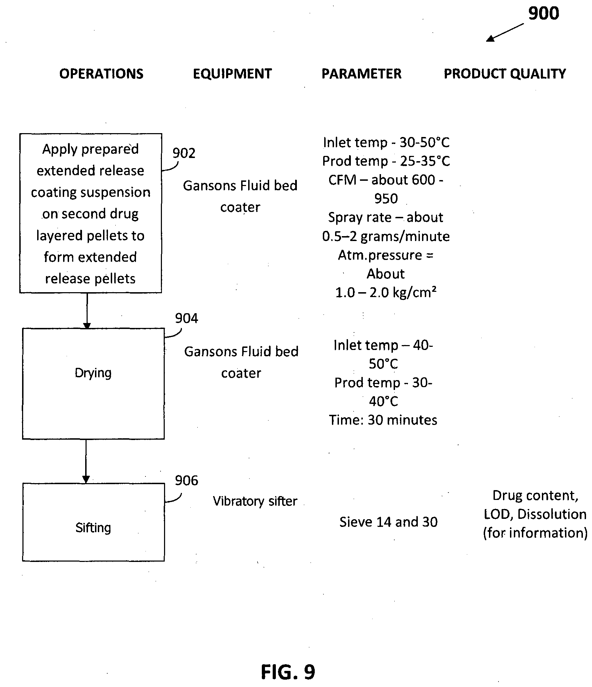

Now referring again to FIG. 1, at step 116, the process 100 comprises performing an extended release coating in the fluid bed coater by applying extended release coating suspension on the second drug layered pellets to form extended release pellets. Referring to 902, 904 and 906 of FIG. 9, the extended release pellets is formed by applying the extended release coating suspension formed at 808 of FIG. 8 on the second drug layered pellets formed at 706 of FIG. 7 to form extended release pellets. Specifically, at 902, the extended release coating suspension formed at 808 of FIG. 8 is applied on the second drug layered pellets formed at 706 of FIG. 7 in fluid bed coater at predetermined parameters to form extended release pellets. As used herein, predetermined parameters include an inlet temperature of about 30°C to about 50°C, product temperature of about 25°C to about 35°C, fluidization of air volume of about 600 CFM to about 950 CFM, spraying rate of the seal coating suspension of about 0.5 grams to about 2 grams per minute and atmospheric pressure of about 1.0 kg/cm² to about 2.0 kg/cm². Although particular predetermined parameters are mentioned above, it will be evident to a person skilled in the art to modify predetermined parameters based on obvious variations.

At 904, the extended release pellets formed at 902 is dried in the fluid bed coater for 30 minutes at predetermined parameters. As used herein, predetermined parameters include an inlet temperature of about 40°C to about 50°C, product temperature of about 30°C to about 40°C. Although particular predetermined parameters are mentioned above, it will be evident to a person skilled in the art to modify predetermined parameters based on obvious variations. The dried extended release pellets may have loss on drying (LOD) of not more than 2 percent.

At 906, the dried extended release pellets are sifted/sieved in the vibratory sifter of about ASTM 14 to about ASTM 30 to form the dried and sieved extended release pellets.

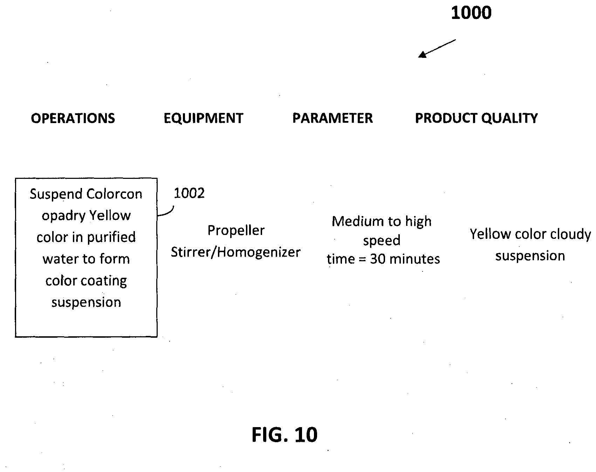

Now referring again to FIG. 1, at step 118, the process 100 comprises preparing a color coating suspension. Referring to 1002 of FIG. 10, the color coating suspension is formed by mixing the colorcon opadry yellow color in to the purified water. Specifically, at 1002 colorcon opadry yellow color is suspended in purified water under stirring/ homogenizing conditions in a propeller stirrer/homogenizer from medium to high speed for about 30 minutes or till yellow color cloudy suspension is formed. This yellow color cloudy suspension formed at 606 is the color coating suspension.

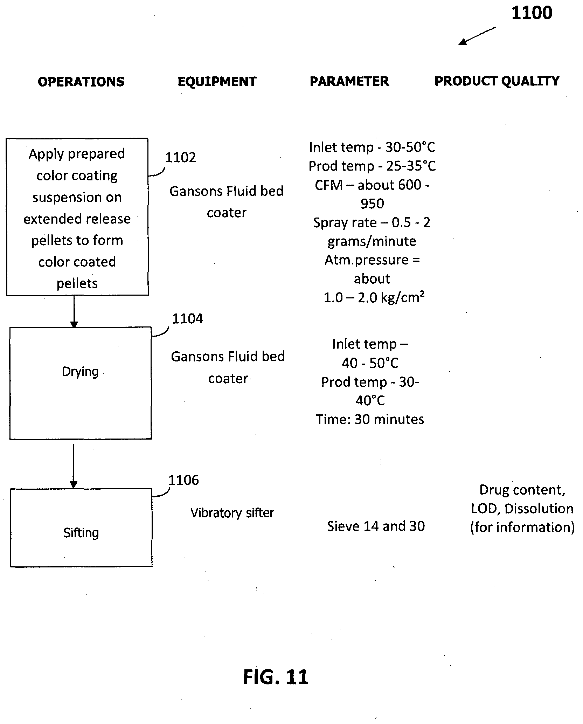

Now referring again to FIG. 1, at step 120, the process 100 comprises performing a color coating in the fluid bed coater by applying the color coating suspension on the extended release pellets to form color coated pellets. Referring to 1102, 1104 and 1106 of FIG. 11, the color coated pellets is formed by applying the color coating suspension formed at 1002 of FIG. 10 on extended release pellets formed at 906 of FIG. 9. Specifically, at 1102, the color coating suspension formed at 1002 of FIG. 10 is applied on extended release pellets formed at 906 of FIG. 9 in fluid bed coater at predetermined parameters to form color coated pellets. As used herein, predetermined parameters include an inlet temperature of about 30°C to about 50°C, product temperature of about 25°C to about 35°C, fluidization of air volume of about 600 CFM to about 950 CFM, spraying rate of the first drug layering suspension of about 0.5 grams to about 2 grams per minute and atmospheric pressure of about 1.0 kg/cm² to about 2.0 kg/cm². Although particular predetermined parameters are mentioned above, it will be evident to a person skilled in the art to modify the predetermined parameters based on obvious variations.

At 1104, the color coated pellets formed at 702 is dried in the fluid bed coater for 30 minutes at predetermined parameters. As used herein, predetermined parameters include an inlet temperature of about 40°C to about 50°C, product temperature of about 30°C to about 40°C. Although particular predetermined parameters are mentioned above, it will be evident to a person skilled in the art to modify predetermined parameters based on obvious variations. The dried color coated pellets may have loss on drying (LOD) of not more than 2 percent.

At 1106, the dried color coated pellets are sifted/sieved in the vibratory sifter of about ASTM 14 to about ASTM 30 to form the dried and sieved color coated pellets.

Now referring again to FIG. 1, at step 122, the process 100 comprises lubricating the color coated pellets to form mesalamine pellets. Referring to 1202 of FIG. 12, the mesalamine pellets is formed by lubricating the color coated pellets. Specifically, at 1202 the color coated pellets formed at 1106 of FIG. 11 is lubricated with talcum in coating pan at slow speed of 8rpm for 3 minutes to form mesalamine pellets.

The process may further comprise packing the mesalamine pellets formed at 1202 of FIG. 12. In an embodiment, the packing is done using Aluminium-Aluminium paper coated sachets equivalent to 500 miligrams, 1grams and 2grams compositions.

Without in any way limiting scope, interpretation, or application of the claims appearing below, advantages of one or more of the exemplary embodiments disclosed herein include seal coating which reduces the quantity of the extended release coat.; and further, mesalamine pellets as described herein also controls the release pattern after 8 hours of release. Further, mesalamine pellets as described herein reduces dosing frequency and has better therapeutic effect. Further, mesalamine pellets as described herein have better patient compliance.

The description of the process for preparation of mesalamine composition 1 of the present invention is further illustrated by the following nonlimiting example. However, a person skilled in the art would recognize that, the specific example is intended to illustrate, not limit, the scope of the present invention.

### EXAMPLE 1

In Example 1, a process for preparing mesalamine pellets is described. The process for preparing mesalamine pellets is initiated by preparing the first drug layering suspension. The first drug layering suspension is prepared by mixing 88.76 grams (g) of methocel E3 (HPMC 603) in to 1133g of hot purified water (about 60°C) under stirring/ homogenizing conditions in a propeller stirrer/homogenizer from medium to fast speed for about 30 minutes or till clear solution is formed. Thereafter, 6.3g of simethicone 30% emulsion (solids 1.89g) and 3.15g of polysorbate 80 is added in to above methocel E3 solution under stirring/ homogenizing conditions in a propeller stirrer/homogenizer from medium to fast speed for about 15 minutes and cooled to reach room temperature to form cloudy suspension. Further, 295.86g of mesalamine is added in to above suspension under stirring/ homogenizing conditions in a propeller stirrer/homogenizer from medium to fast speed for about 60 minutes or till light cream color suspension is formed. The light cream suspension formed is the first drug layering suspension.

Thereafter, first drug layering is performed by applying the first drug layering suspension on 350g of sugar spheres of the size about 300µm to about 500µm in fluid bed coater at pre-determined parameters to form first drug layered pellets. As used herein, pre-determined parameters include an inlet temperature of about 30°C to about 50°C, product temperature of about 25°C to about 35°C, fluidization of air volume of about 500 CFM, spraying rate of the first drug layering suspension of about 0.8 grams to about 2 grams per minute and atmospheric pressure of about 0.5 kg/cm² to about 1.5 kg/cm². Further, the first drug layered pellets is dried in the fluid bed coater for 30 minutes at the pre-determined parameters. As used herein, pre-determined parameters include an inlet temperature of about 40°C to about 50°C, and product temperature of about 30°C to about 40°C. After which the dried first drug layered pellets are sifted/sieved in the vibratory sifter of about ASTM 18 to about ASTM 30 to form the dried and sieved first drug layered pellets. Theoretical weight of the first drug layered pellets is 739.66g while practical weight of the first drug layered pellets is 718.95g giving yield of 97.2%.

The first drug layered pellets formed in the process described above have an appearance of light to dark beige spherical pellets. Theoretical assay of the first drug layered pellets is 400 mg/g while the practical assay of the first drug layered pellets is 400.71 mg/g having % yield of 100.18.

In TABLE 1A below, the different ingredients to prepare first drug layering suspension and performing first drug layering are depicted. Further, TABLE 1B below shows the analytical results of the first drug layered pellets.

**TABLE 1A**

| Ingredients | Qty. in (g) |
|---|---|
| Sugar spheres | 350.0 |
| (300 µm - 500 µm) | |
| Mesalamine | 295.86 |
| HPMC 603 | 88.76 |
| Simethicone 30% | 6.3 |
| | (Solids 1.89) |
| Tween / Polysorbate 80 | 3.15 |
| Purified water* | 1133.0 |
| | |
| Theoretical weight | 739.66 |
| Practical weight | 718.95 |
| % Yield | 97.2 |

**TABLE 1B**

| Appearance | Light to dark beige spherical pellets |
|---|---|
| Theoretical assay | 400 mg/g |
| Practical assay | 400.71 mg/g |
| % Yield | 100.18 |

Next , seal coating suspension is formed by dissolving 13.5g of ethocel N 20 (ethyl cellulose N20) in 350.85g of isopropyl alcohol under stirring/homogenizing conditions in propeller stirrer/homogenizer from medium to high speed for about 30 minutes for a clear to cloudy solution is formed. Thereafter, 9.0g of povidone K 30 is added in to above ethocel N 20 solution under stirring/homogenizing conditions in propeller stirrer/homogenizer from medium to high speed for about 15 minutes or till clear to cloudy suspension is formed. Next, 115.84g of purified water is added in to above solution under stirring/ homogenizing conditions in propeller stirrer/homogenizer from medium to high speed for about 10 minutes or till clear to cloudy suspension is formed. Thereafter, 2.48g of dibutyl sebacate and 5.03g of purified talc (talcum) is added in to above solution under stirring/ homogenizing conditions in propeller stirrer/homogenizer from medium to high speed for about 30 minutes or till clear to cloudy suspension is formed. This suspension formed is the seal coating suspension.

Next, seal coating is performed by applying the seal coating suspension on 600g of first drug layered pellets in fluid bed coater at predetermined parameters to form seal coated pellets. As used herein, predetermined parameters include an inlet temperature of about 30°C to about 50°C, product temperature of about 25°C to about 35°C, fluidization of air volume of about 600 CFM, spraying rate of the seal coating suspension of about 0.5 grams to about 2 grams per minute and atmospheric pressure of about 1.0 kg/cm² to about 2.0 kg/cm². Further, the seal coated pellets formed are dried in the fluid bed coater for 30 minutes at predetermined parameters. As used herein, predetermined parameters include an inlet temperature of about 40°C to about 50°C, product temperature of about 30°C to about 40°C. Thereafter, the dried seal coated pellets are sifted/sieved in the vibratory sifter of about ASTM 18 to about ASTM 30 to form the dried and sieved seal coated pellets. Theoretical weight of the seal coated pellets is 630.0g while practical weight of the first drug layered pellets is 624.0g giving the yield of 99.0%.

The seal coated pellets formed in the process described above have an appearance of light to dark beige spherical pellets. Theoretical assay of the seal coated pellets is 381 mg/g while the practical assay of the seal coated pellets is 380.83 mg/g having % yield of 95.95.

In TABLE 1C below, the different ingredients to prepare seal coating suspension and performing seal coating are depicted. Further, TABLE 1D below shows the analytical results of the seal coated pellets.

**TABLE 1C**

| Ingredients | Qty. in (g) |
|---|---|
| Mesalamine drug pellets (first drug layered pellets) | 600 |
| Ethyl cellulose N20 | 13.5 |
| PVP K-30 | 9.0 |
| Dibutyl sebaccate | 2.48 |
| Talcum | 5.03 |
| Isopropyl alcohol* | 350.85 |
| Purified water* | 115.84 |
| | |
| Theoretical weight | 630.0 |
| Practical weight | 624.0 |
| % Yield | 99.0 |

**TABLE 1D**

| Appearance | Light to dark beige spherical pellets |
|---|---|
| Theoretical assay | 381 mg/g |
| Practical assay | 380.83 mg/g |
| % Yield | 95.95 % |

Thereafter, second drug layering suspension is prepared by dissolving 544.08g of methocel E3(HPMC 603) in to 6946.13g of hot purified water (about 60°C) under stirring/ homogenizing conditions in a propeller stirrer/homogenizer from medium to fast speed for about 30 minutes or till clear solution is formed. Thereafter, 35.7g of simethicone 30% emulsion (solid 10.7g) and 18.0g of polysorbate 80 (tween 80) is added in to above methocel E3 solution under stirring/ homogenizing conditions in a propeller stirrer/homogenizer from medium to fast speed for about 15 minutes and cooled to reach room temperature to form cloudy suspension. Further, 1813.61g of mesalamine is added in to above suspension under stirring/ homogenizing conditions in a propeller stirrer/homogenizer from medium to fast speed for about 60 minutes or till light cream color suspension is formed. The light cream suspension formed is the second drug layering suspension.

Thereafter, second drug layering is performed by applying the second drug layering suspension on 600g of seal coated pellets in fluid bed coater at predetermined parameters to form second drug layered pellets. As used herein, predetermined parameters parameters include an inlet temperature of about 30°C to about 50°C, product temperature of about 25°C to about 35°C, fluidization of air volume of about 600 CFM to about 950 CFM, spraying rate of the first drug layering suspension of about 1.0 grams to about 5 grams per minute and atmospheric pressure of about 1.0 kg/cm² to about 2.0 kg/cm². Further, the second drug layered pellets is dried in the fluid bed coater for 30 minutes at predetermined parameters. As used herein, predetermined parameters include an inlet temperature of about 40°C to about 60°C, product temperature of about 30°C to about 40°C. Next, the dried first drug layered pellets are sifted/sieved in the vibratory sifter of about ASTM 14 to about ASTM 30 to form the dried and sieved second drug layered pellets. Theoretical weight of the second drug layered pellets is 2986.4g while practical weight of the second drug layered pellets is 2891.7g giving the yield of 96.8%.

The second drug layered pellets formed in the process described above have an appearance of light to dark beige spherical pellets. Theoretical assay of the second drug layered pellets is 683.8 mg/g while the practical assay of the second drug layered pellets is 653.54 mg/g having % yield of 95.57.

In TABLE 1E below, the different ingredients to prepare second drug layering suspension and performing second drug layering are depicted. Further, TABLE 1F below shows the analytical results of the second drug layered pellets.

**TABLE 1E**

| Ingredients | Qty. in (g) |
|---|---|
| Seal coated pellets | 600 |
| Mesalamine | 1813.61 |
| HPMC 603 | 544.08 |
| Simethicone 30% | 35.7 |
| | (Solid 10.7) |
| Tween / Polysorbate 80 | 18.0 |
| Purified water* | 6946.13 |
| | |
| Theoretical weight | 2986.4 |
| Practical weight | 2891.7 |
| % Yield | 96.8 |

**TABLE 1F**

| Appearance | Light to dark beige spherical pellets |
|---|---|
| Theoretical assay | 683.8 mg/g |
| Practical assay | 653.54 mg/g |
| % Yield | 95.57 % |

Next, extended release coating suspension is formed by dissolving 23.4g of ethocel N 20 (ethyl cellulose N20) in to 608.13g of isopropyl alcohol under stirring/ homogenizing conditions in a propeller stirrer/homogenizer from medium to high speed for about 30 minutes or till cloudy suspension is formed. Therafter, 15.63g of povidone K 30(PVP K-30) is added in to above ethocel N 20 solution under stirring/ homogenizing conditions in a propeller stirrer/homogenizer from medium to high speed for about 15 minutes or till clear to cloudy suspension is formed. Further, 200.77g of purified water is added in to above solution under stirring/ homogenizing conditions in a propeller stirrer/homogenizer from medium to high speed for about 10 minutes or till clear to cloudy suspension is formed. Next, 4.28g of dibutyl sebacate and 8.69g of purified talc is added in to above solution under stirring/ homogenizing conditions in a propeller stirrer/homogenizer from medium to high speed for about 15 minutes or till clear to cloudy suspension is formed. This cloudy suspension formed is the extended release coating suspension.

Next, extended release coating is performed by applying the extended release coating suspension on 650g of second drug layered pellets in fluid bed coater at predetermined parameters to form extended release pellets. As used herein, predetermined parameters include an inlet temperature of about 30°C to about 50°C, product temperature of about 25°C to about 35°C, fluidization of air volume of about 600 CFM to about 950 CFM, spraying rate of the seal coating suspension of about 0.5 grams to about 2 grams per minute and atmospheric pressure of about 1.0 kg/cm² to about 2.0 kg/cm². Further, the extended release pellets is dried in the fluid bed coater for 30 minutes at predetermined parameters. As used herein, predetermined parameters include an inlet temperature of about 40°C to about 50°C, product temperature of about 30°C to about 40°C. Next, the dried extended release pellets are sifted/sieved in the vibratory sifter of about ASTM 14 to about ASTM 30 to form the dried and sieved extended release pellets. The total % of polymer applied to control the release pattern of the second drug layered pellets is 8%. Theoretical weight of the extended release pellets is 702.0g while practical weight of the extended release pellets is 695.85g giving the yield of 99.12%.

The extended release pellets in the process described above have an appearance of light to dark beige spherical pellets. The mean assay (practical) of the extended release pellets is 599.0mg/g.

In TABLE 1G below, the different ingredients to prepare extended release coating suspension and performing extended release coating are depicted. Further, TABLE 1H below shows the analytical results of the extended release pellets.

**TABLE 1G**

| Ingredients | Qty. in (g) |
|---|---|
| Total % of polymer applied | 8 |
| second drug layered pellets | 650 |
| Ethyl cellulose N20 | 23.4 |
| PVP K-30 | 15.63 |
| Dibutyl sebaccate | 4.28 |
| Talcum | 8.69 |
| Isopropyl alcohol* | 608.13 |
| Purified water* | 200.77 |
| | |
| Theoretical weight | 702.0 |
| Practical weight | 695.85 |
| % Yield | 99.12% |

**TABLE 1H**

| Appearance | | Light to dark beige spherical pellets |
|---|---|---|
| Mean assay (practical) | | 599.0 mg/gm |
| Dissolution Paddle 100 RPM. 900 ml of Phosphate buffer pH 7.5 | 1 Hr | 19.1 |
| | (NMT 30%) | |
| | 2 Hr | 35.6 |
| | (20% - 60 %) | |
| | 4 Hr | 63.9 |
| | (40% - 80 %) | |
| | 8 Hr | 84.4 |
| | (NLT 75%) | |

Next, the color coating suspension is formed by suspending 30g of colorcon opadry yellow color in 270g of purified water under stirring/ homogenizing conditions in a propeller stirrer/homogenizer from medium to high speed for about 30 minutes or till yellow color cloudy suspension is formed. This yellow color cloudy suspension formed is the color coating suspension.

Thereafter, color coating is performed by applying the color coating suspension on 600g of extended release pellets in fluid bed coater at predetermined parameters to form color coated pellets. As used herein, predetermined parameters include an inlet temperature of about 30°C to about 50°C, product temperature of about 25°C to about 35°C, fluidization of air volume of about 600 CFM to about 950 CFM, spraying rate of the first drug layering suspension of about 0.5 grams to about 2 grams per minute and atmospheric pressure of about 1.0 kg/cm² to about 2.0 kg/cm². Further, the color coated pellets formed are dried in the fluid bed coater for 30 minutes at predetermined parameters. As used herein, predetermined parameters include an inlet temperature of about 40°C to about 50°C, product temperature of about 30°C to about 40°C. Next, the dried color coated pellets are sifted/sieved in the vibratory sifter of about ASTM 14 to about ASTM 30 to form the dried and sieved color coated pellets. Theoretical weight of the color coated pellets is 630g while practical weight of the color coated pellets is 619.0g giving the yield of 98.25%.

The color coated pellets in the process described above have an appearance of yellow to yellowish spherical pellets. The mean assay (practical) of the color coated pellets is 556.8mg/g.

In TABLE 1I below, the different ingredients to prepare color coating suspension and performing color coating are depicted. Further, TABLE 1J below shows the analytical results of the color coated pellets.

**TABLE 1I**

| Ingredients | Qty. in (g) |
|---|---|
| Extended release pellets | 600 |
| Instacoat / Colorcon coloring material | 30 |
| Purified water | 270.0 |
| | |
| Theoretical weight | 630 |
| Practical weight | 619.0 |
| % Yield | 98.25 % |

**TABLE 1J**

| Appearance | | Yellow to yellowish spherical pellets |
|---|---|---|
| Mean assay (practical) | | 556.8 mg/gm |
| Dissolution Paddle 100 RPM. 900 ml of Phosphate buffer pH 7.5 | 1 Hr | 21.0 |
| | (NMT 30%) | |
| | 2 Hr | 38.6 |
| | (20% - 60 %) | |
| | 4 Hr | 62.5 |
| | (40% - 80 %) | |
| | 8 Hr | 86.5 |
| | (NLT 75%) | |

Thereafter, , the color coated pellets formed above is lubricated with about 0.5% talcum in coating pan at slow speed of 8rpm for 3 minutes to form mesalamine pellets.

### EXAMPLE 2

In Example 2, yet another example of a process for preparing a mesalamine pellets is described. The process for preparing mesalamine pellets is initiated by preparing the first drug layering suspension. The first drug layering suspension is prepared by mixing 126.8 grams (g) of methocel E3(HPMC 603) in to 1618.8g of hot purified water (about 60°C) under stirring/ homogenizing conditions in a propeller stirrer/homogenizer from medium to fast speed for about 30 minutes or till clear solution is formed. Thereafter, 9.0g of simethicone 30% emulsion (solids 2.7g) and 4.5g of polysorbate 80 is added in to above methocel E3 solution under stirring/ homogenizing conditions in a propeller stirrer/homogenizer from medium to fast speed for about 15 minutes and cooled to reach room temperature to form cloudy suspension. Further, 422.66g of mesalamine is added in to above suspension under stirring/ homogenizing conditions in a propeller stirrer/homogenizer from medium to fast speed for about 60 minutes or till light cream color suspension is formed. The light cream suspension formed is the first drug layering suspension.

Thereafter, first drug layering is performed by applying the first drug layering suspension on 500g of sugar spheres of the size about 300µm to about 500µm in fluid bed coater at pre-determined parameters to form first drug layered pellets. As used herein, pre-determined parameters include an inlet temperature of about 30°C to about 50°C, product temperature of about 25°C to about 35°C, fluidization of air volume of about 500 CFM, spraying rate of the first drug layering suspension of about 0.8 grams to about 2 grams per minute and atmospheric pressure of about 0.5 kg/cm² to about 1.5 kg/cm². Further, the first drug layered pellets is dried in the fluid bed coater for 30 minutes at the pre-determined parameters. As used herein, pre-determined parameters include an inlet temperature of about 40°C to about 50°C, and product temperature of about 30°C to about 40°C. After which the dried first drug layered pellets are sifted/sieved in the vibratory sifter of about ASTM 18 to about ASTM 30 to form the dried and sieved first drug layered pellets. Theoretical weight of the first drug layered pellets is 1056.66g while practical weight of the first drug layered pellets is 1033.4g giving the yield of 97.8%.

The first drug layered pellets formed in the process described above have an appearance of light to dark beige spherical pellets. Theoretical assay of the first drug layered pellets is 400 mg/g while the practical assay of the first drug layered pellets is 395.9 mg/g having % yield of 98.97.

In TABLE 2A below, the different ingredients to prepare first drug layering suspension and performing first drug layering are depicted. Further, TABLE 2B below shows the analytical results of the first drug layered pellets.

**TABLE 2A**

| Ingredients | Qty. in (g) |
|---|---|
| Sugar spheres | 500 |
| (300 - 500 µm) | |
| Mesalamine | 422.66 |
| HPMC 603 | 126.8 |
| Simethicone 30% | 9.0 |
| | (Solids 2.7) |
| Tween / Polysorbate 80 | 4.5 |
| Purified water* | 1618.8 |
| | |
| Theoretical weight | 1056.66 |
| Practical weight | 1033.4 |
| % Yield | 97.8 |

**TABLE 2B**

| Appearance | Light to dark beige spherical pellets |
|---|---|
| Theoretical assay | 400 mg/g |
| Practical assay | 395.9 mg/g |
| % Yield | 98.97 |

Next, seal coating suspension is formed by dissolving 14.62g of ethocel N 20 (ethyl cellulose N20) in 380.12g of isopropyl alcohol under stirring/homogenizing conditions in propeller stirrer/homogenizer from medium to high speed for about 30 minutes for a clear to cloudy solutionis formed. Thereafter, 9.75g of povidone K 30 is added in to above ethocel N 20 solution under stirring/homogenizing conditions in propeller stirrer/homogenizer from medium to high speed for about 15 minutes or till clear to cloudy suspension is formed. Next, 125.5g of purified water is added in to above solution under stirring/ homogenizing conditions in propeller stirrer/homogenizer from medium to high speed for about 10 minutes or till clear to cloudy suspension is formed. Thereafter, 2.68g of dibutyl sebacate and 5.46g of purified talc (talcum) is added in to above solution under stirring/ homogenizing conditions in propeller stirrer/homogenizer from medium to high speed for about 30 minutes or till clear to cloudy suspension is formed. This suspension formed is the seal coating suspension.

Next, seal coating is performed by applying the seal coating suspension on 650g of first drug layered pellets in fluid bed coater at predetermined parameters to form seal coated pellets. As used herein, predetermined parameters include an inlet temperature of about 30°C to about 50°C, product temperature of about 25°C to about 35°C, fluidization of air volume of about 600 CFM, spraying rate of the seal coating suspension of about 0.5 grams to about 2 grams per minute and atmospheric pressure of about 1.0 kg/cm² to about 2.0 kg/cm². Further, the seal coated pellets formed are dried in the fluid bed coater for 30 minutes at predetermined parameters. As used herein, predetermined parameters include an inlet temperature of about 40°C to about 50°C, product temperature of about 30°C to about 40°C. Thereafter, the dried seal coated pellets are sifted/sieved in the vibratory sifter of about ASTM 18 to about ASTM 30 to form the dried and sieved seal coated pellets. Theoretical weight of the seal coated pellets is 682.5g while practical weight of the first drug layered pellets is 676.4g giving the yield of 99.0%.

The seal coated pellets formed in the process described above have an appearance of light to dark beige spherical pellets. Theoretical assay of the seal coated pellets is 381 mg/g while the practical assay of the seal coated pellets is 395.9 mg/g having % yield of 98.97.

In TABLE 2C below, the different ingredients to prepare seal coating suspension and performing seal coating are depicted. Further, TABLE 2D below shows the analytical results of the seal coated pellets.

**TABLE 2C**

| Ingredients | Qty. in (g) |
|---|---|
| Mesalamine drug pellets (first drug layered pellets) | 650 |
| Ethyl cellulose N20 | 14.62 |
| PVP K-30 | 9.75 |
| Dibutyl sebaccate | 2.68 |
| Talcum | 5.46 |
| Isopropyl alcohol* | 380.12 |
| Purified water* | 125.5 |
| | |
| Theoretical weight | 682.5 |
| Practical weight | 676.4 |
| % Yield | 99.0 % |

**TABLE 2D**

| Appearance | Light to dark beige spherical pellets |
|---|---|
| Theoretical assay | 381 mg/g |
| Practical assay | 395.9 mg/g |
| % Yield | 98.97 |

Thereafter, second drug layering suspension is prepared by dissolving 453.4g of methocel E3(HPMC 603) in to 5788.47g of hot purified water (about 60°C) under stirring/ homogenizing conditions in a propeller stirrer/homogenizer from medium to fast speed for about 30 minutes or till clear solution is formed. Thereafter, 29.7g of simethicone 30% emulsion (solid 8.91g) and 15.0g of polysorbate 80 (tween 80) is added in to above methocel E3 solution under stirring/ homogenizing conditions in a propeller stirrer/homogenizer from medium to fast speed for about 15 minutes and cooled to reach room temperature to form cloudy suspension. Further, 1511.35g of mesalamine is added in to above suspension under stirring/ homogenizing conditions in a propeller stirrer/homogenizer from medium to fast speed for about 60 minutes or till light cream color suspension is formed. The light cream suspension formed is the second drug layering suspension.

Thereafter, second drug layering is performed by applying the second drug layering suspension on 500g of seal coated pellets in fluid bed coater at predetermined parameters to form second drug layered pellets. As used herein, predetermined parameters include an inlet temperature of about 30°C to about 50°C, product temperature of about 25°C to about 35°C, fluidization of air volume of about 600 CFM to about 950 CFM, spraying rate of the first drug layering suspension of about 1.0 grams to about 5 grams per minute and atmospheric pressure of about 1.0 kg/cm² to about 2.0 kg/cm². Further, the second drug layered pellets is dried in the fluid bed coater for 30 minutes at predetermined parameters. As used herein, predetermined parameters include an inlet temperature of about 40°C to about 60°C, product temperature of about 30°C to about 40°C. Next, the dried first drug layered pellets are sifted/sieved in the vibratory sifter of about ASTM 14 to about ASTM 30 to form the dried and sieved second drug layered pellets. Theoretical weight of the second drug layered pellets is 2488.66g while practical weight of the second drug layered pellets is 2418.9g giving the yield of 97.2%.

The second drug layered pellets formed in the process described above have an appearance of light to dark beige spherical pellets. Theoretical assay of the second drug layered pellets is 683.8 mg/g while the practical assay of the second drug layered pellets is 688.6 mg/g having % yield of 100.7.

In TABLE 2E below, the different ingredients to prepare second drug layering suspension and performing second drug layering are depicted. Further, TABLE 2F below shows the analytical results of the second drug layered pellets.

**TABLE 2E**

| **Ingredients** | **Qty. in (g)** |
|---|---|
| Seal coated pellets | 500 |
| Mesalamine | 1511.35 |
| HPMC 603 | 453.4 |
| Simethicone 30% | 29.7 |
| | (Solid 8.91) |
| Tween / Polysorbate 80 | 15.0 |
| Purified water* | 5788.47 |
| | |
| Theoretical weight | 2488.66 |
| Practical weight | 2418.9 |
| % Yield | 97.2 |

**TABLE 2F**

| Appearance | Light to dark beige spherical pellets |
|---|---|
| Theoretical assay | 683.8 mg/g |
| Practical assay | 688.6 mg/g |
| % Yield | 100.7 % |

Next, extended release coating suspension is formed by dissolving 9.46g of ethocel N 20 (ethyl cellulose N20) in to 245.63g of isopropyl alcohol under stirring/ homogenizing conditions in a propeller stirrer/homogenizer from medium to high speed for about 30 minutes or till cloudy suspension is formed. Thereafter, 6.31g of povidone K 30(PVP K-30) is added in to above ethocel N 20 solution under stirring/ homogenizing conditions in a propeller stirrer/homogenizer from medium to high speed for about 15 minutes or till clear to cloudy suspension is formed. Further, 81.09g of purified water is added in to above solution under stirring/ homogenizing conditions in a propeller stirrer/homogenizer from medium to high speed for about 10 minutes or till clear to cloudy suspension is formed. Next, 1.75g of dibutyl sebacate and 3.50g of purified talc is added in to above solution under stirring/ homogenizing conditions in a propeller stirrer/homogenizer from medium to high speed for about 15 minutes or till clear to cloudy suspension is formed. This cloudy suspension formed is the extended release coating suspension.

Next, extended release coating is performed by applying the extended release coating suspension on 350g of second drug layered pellets in fluid bed coater at predetermined parameters to form extended release pellets. As used herein, predetermined parameters include an inlet temperature of about 30°C to about 50°C, product temperature of about 25°C to about 35°C, fluidization of air volume of about 600 CFM to about 950 CFM, spraying rate of the seal coating suspension of about 0.5 grams to about 2 grams per minute and atmospheric pressure of about 1.0 kg/cm² to about 2.0 kg/cm². Further, the extended release pellets is dried in the fluid bed coater for 30 minutes at predetermined parameters. As used herein, predetermined parameters include an inlet temperature of about 40°C to about 50°C, product temperature of about 30°C to about 40°C. Next, the dried extended release pellets are sifted/sieved in the vibratory sifter of about ASTM 14 to about ASTM 30 to form the dried and sieved extended release pellets. The total % of polymer applied to control the release pattern of the second drug layered pellets is 6%. Theoretical weight of the extended release pellets is 371.00g while practical weight of the extended release pellets is 369.00g giving the yield of 99.4%.

The extended release pellets in the process described above have an appearance of light to dark beige spherical pellets. The mean assay (practical) of the extended release pellets is 644.48mg/g.

In TABLE 2G below, the different ingredients to prepare extended release coating suspension and performing extended release coating are depicted. Further, TABLE 2H below shows the analytical results of the extended release pellets.

**TABLE 2G**

| Ingredients | Qty. in (g) |
|---|---|
| Total % of polymer applied | 6% |
| second drug layered pellets | 350 |
| Ethyl cellulose N20 | 9.46 |
| PVP K-30 | 6.31 |
| Dibutyl sebaccate | 1.75 |
| Talcum | 3.50 |
| Isopropyl alcohol | 245.63 |
| Purified water | 81.09 |
| | |
| Theoretical weight | 371.00 |
| Practical weight | 369.00 |
| % Yield | 99.4 % |

**TABLE 2H**

| Appearance | | Light to dark beige spherical pellets |
|---|---|---|
| Mean assay (practical) | | 644.48 mg/gm |
| Dissolution Paddle 100 RPM. 900 ml of Phosphate buffer pH 7.5 | 1 Hr | 21.9 |
| | (NMT 30%) | |
| | 2 Hr | 37.1 |
| | (20% - 60 %) | |
| | 4 Hr | 68.9 |
| | (40% - 80 %) | |
| | 8 Hr | 93.5 |
| | (NLT 75%) | |

Next, the color coating suspension is formed by suspending 16.25g of colorcon opadry yellow color in 146.25g of purified water under stirring/ homogenizing conditions in a propeller stirrer/homogenizer from medium to high speed for about 30 minutes or till yellow color cloudy suspension is formed. This yellow color cloudy suspension formed is the color coating suspension.

Thereafter, color coating is performed by applying the color coating suspension on 325g of extended release pellets in fluid bed coater at predetermined parameters to form color coated pellets. As used herein, predetermined parameters include an inlet temperature of about 30°C to about 50°C, product temperature of about 25°C to about 35°C, fluidization of air volume of about 600 CFM to about 950 CFM, spraying rate of the first drug layering suspension of about 0.5 grams to about 2 grams per minute and atmospheric pressure of about 1.0 kg/cm² to about 2.0 kg/cm². Further, the color coated pellets formed are dried in the fluid bed coater for 30 minutes at predetermined parameters. As used herein, predetermined parameters include an inlet temperature of about 40°C to about 50°C, product temperature of about 30°C to about 40°C. Next, the dried color coated pellets are sifted/sieved in the vibratory sifter of about ASTM 14 to about ASTM 30 to form the dried and sieved color coated pellets. Theoretical weight of the color coated pellets is 341.25g while practical weight of the color coated pellets is 337.57g giving the yield of 98.92%.

The color coated pellets in the process described above have an appearance of yellow to yellowish spherical pellets. The mean assay (practical) of the color coated pellets is 616.5mg/g.

In TABLE 21 below, the different ingredients to prepare color coating suspension and performing color coating are depicted. Further, TABLE 2J below shows the analytical results of the color coated pellets.

**TABLE 2I**

| Ingredients | Qty. in (g) |
|---|---|
| Extended release pellets | 325 |
| Instacoat / Colorcon coloring material | 16.25 |
| Purified water | 146.25 |
| | |
| Theoretical weight | 341.25 |
| Practical weight | 337.57 |
| % Yield | 98.92 % |

**TABLE 2J**

| Appearance | | Yellow to yellowish spherical pellets |
|---|---|---|
| Mean assay (practical) | | 616.5 mg/gm |
| Dissolution Paddle 100 RPM. 900 ml of Phosphate buffer pH 7.5 | 1 Hr | 23.8 |
| | (NMT 30%) | |
| | 2 Hr | 40.3 |
| | (20% - 60 %) | |
| | 4 Hr | 71.3 |
| | (40% - 80 %) | |
| | 8 Hr | 103.1 |
| | (NLT 75%) | |

Thereafter, the color coated pellets formed above is lubricated with about 0.5% talcum in coating pan at slow speed of 8rpm for 3 minutes to form mesalamine pellets.

### EXAMPLE 3

Yet another example of a process for preparing a mesalamine pellets is described herein example 3. The process for preparing mesalamine pellets is initiated by preparing the first drug layering suspension. The first drug layering suspension is prepared by mixing 126.8 grams (g) of methocel E3(HPMC 603) in to 1618.8g of hot purified water (about 60°C) under stirring/ homogenizing conditions in a propeller stirrer/homogenizer from medium to fast speed for about 30 minutes or till clear solution is formed. Thereafter, 9.0g of simethicone 30% emulsion (solids 2.7g) and 4.5g of polysorbate 80 is added in to above methocel E3 solution under stirring/ homogenizing conditions in a propeller stirrer/homogenizer from medium to fast speed for about 15 minutes and cooled to reach room temperature to form cloudy suspension. Further, 422.66g of mesalamine is added in to above suspension under stirring/ homogenizing conditions in a propeller stirrer/homogenizer from medium to fast speed for about 60 minutes or till light cream color suspension is formed. The light cream suspension formed is the first drug layering suspension.

Thereafter, first drug layering is performed by applying the first drug layering suspension on 500g of sugar spheres of the size about 300µm to about 500µm in fluid bed coater at pre-determined parameters to form first drug layered pellets. As used herein, pre-determined parameters include an inlet temperature of about 30°C to about 50°C, product temperature of about 25°C to about 35°C, fluidization of air volume of about 500 CFM, spraying rate of the first drug layering suspension of kg/cm² to about 1.5 kg/cm². Further, the first drug layered pellets is dried in the fluid bed coater for 30 minutes at the pre-determined parameters. As used herein, pre-determined parameters include an inlet temperature of about 40°C to about 50°C, and product temperature of about 30°C to about 40°C. After which the dried first drug layered pellets are sifted/sieved in the vibratory sifter of about ASTM 18 to about ASTM 30 to form the dried and sieved first drug layered pellets. Theoretical weight of the first drug layered pellets is 1056.66g while practical weight of the first drug layered pellets is 1036.6g giving the yield of 98.1%.

The first drug layered pellets formed in the process described above have an appearance of light to dark beige spherical pellets. Theoretical assay of the first drug layered pellets is 400 mg/g while the practical assay of the first drug layered pellets is 384.0 mg/g having % yield of 96.

In TABLE 3A below, the different ingredients to prepare first drug layering suspension and performing first drug layering are depicted. Further, TABLE 3B below shows the analytical results of the first drug layered pellets.

**TABLE 3A**

| Ingredients | Qty. in (g) |
|---|---|
| Sugar spheres | 500 |
| (300 - 500 µm) | |
| Mesalamine | 422.66 |
| HPMC 603 | 126.8 |
| Simethicone 30% | 9.0 |
| | (Solids 2.7) |
| Tween / Polysorbate 80 | 4.5 |
| Purified water* | 1618.8 |
| | |
| Theoretical weight | 1056.66 |
| Practical weight | 1036.6 |
| % Yield | 98.1 |

**TABLE 3B**

| Appearance | Light to dark beige spherical pellets |
|---|---|
| Theoretical assay | 400 mg/g |
| Practical assay | 384.0 mg/g |
| % Yield | 96.0 |

Next, seal coating suspension is formed by dissolving 14.62g of ethocel N 20 (ethyl cellulose N20) in 380.12g of isopropyl alcohol under stirring/homogenizing conditions in propeller stirrer/homogenizer from medium to high speed for about 30 minutes for a clear to cloudy solutionis formed. Thereafter, 9.75g of povidone K 30 is added in to above ethocel N 20 solution under stirring/homogenizing conditions in propeller stirrer/homogenizer from medium to high speed for about 15 minutes or till clear to cloudy suspension is formed. Next, 125.5g of purified water is added in to above solution under stirring/ homogenizing conditions in propeller stirrer/homogenizer from medium to high speed for about 10 minutes or till clear to cloudy suspension is formed. Thereafter, 2.68g of dibutyl sebacate and 5.46g of purified talc (talcum) is added in to above solution under stirring/ homogenizing conditions in propeller stirrer/homogenizer from medium to high speed for about 30 minutes or till clear to cloudy suspension is formed. This suspension formed is the seal coating suspension.

Next, seal coating is performed by applying the seal coating suspension on 650g of first drug layered pellets in fluid bed coater at predetermined parameters to form seal coated pellets. As used herein, predetermined parameters include an inlet temperature of about 30°C to about 50°C, product temperature of about 25°C to about 35°C, fluidization of air volume of about 600 CFM, spraying rate of the seal coating suspension of about 0.5 grams to about 2 grams per minute and atmospheric pressure of about 1.0 kg/cm² to about 2.0 kg/cm². Further, the seal coated pellets formed are dried in the fluid bed coater for 30 minutes at predetermined parameters. As used herein, predetermined parameters include an inlet temperature of about 40°C to about 50°C, product temperature of about 30°C to about 40°C. Thereafter, the dried seal coated pellets are sifted/sieved in the vibratory sifter of about ASTM 18 to about ASTM 30 to form the dried and sieved seal coated pellets. Theoretical weight of the seal coated pellets is 682.5g while practical weight of the first drug layered pellets is 677.0g giving the yield of 99.2%.

The seal coated pellets formed in the process described above have an appearance of light to dark beige spherical pellets. Theoretical assay of the seal coated pellets is 381 mg/g while the practical assay of the seal coated pellets is 384.0 mg/g having % yield of 96.0.

In TABLE 3C below, the different ingredients to prepare seal coating suspension and performing seal coating are depicted. Further, TABLE 3D below shows the analytical results of the seal coated pellets.

**TABLE 3C**

| Ingredients | Qty. in (g) |
|---|---|
| Mesalamine drug pellets (first drug layered pellets) | 650 |
| Ethyl cellulose N20 | 14.62 |
| PVP K-30 | 9.75 |
| Dibutyl sebaccate | 2.68 |
| Talcum | 5.46 |
| Isopropyl alcohol* | 380.12 |
| Purified water* | 125.5 |
| | |
| Theoretical weight | 682.5 |
| Practical weight | 677.0 |
| % Yield | 99.2% |

**TABLE 3D**

| Appearance | Light to dark beige spherical pellets |
|---|---|
| Theoretical assay | 381 mg/g |
| Practical assay | 384.0 mg/g |
| % | 96.0 |

Thereafter, second drug layering suspension is prepared by dissolving 589.42g of methocel E3(HPMC 603) in to 7525.0g of hot purified water (about 60°C) under stirring/ homogenizing conditions in a propeller stirrer/homogenizer from medium to fast speed for about 30 minutes or till clear solution is formed. Thereafter, 38.63g of simethicone 30% emulsion (solid 11.59g) and 19.49g of polysorbate 80 (tween 80) is added in to above methocel E3 solution under stirring/ homogenizing conditions in a propeller stirrer/homogenizer from medium to fast speed for about 15 minutes and cooled to reach room temperature to form cloudy suspension. Further, 1964.75g of mesalamine is added in to above suspension under stirring/ homogenizing conditions in a propeller stirrer/homogenizer from medium to fast speed for about 60 minutes or till light cream color suspension is formed. The light cream suspension formed is the second drug layering suspension.

Thereafter, second drug layering is performed by applying the second drug layering suspension on 650g of seal coated pellets in fluid bed coater at predetermined parameters to form second drug layered pellets. As used herein, predetermined parameters include an inlet temperature of about 30°C to about 50°C, product temperature of about 25°C to about 35°C, fluidization of air volume of about 600 CFM to about 950 CFM, spraying rate of the first drug layering suspension of about 1.0 grams to about 5 grams per minute and atmospheric pressure of about 1.0 kg/cm² to about 2.0 kg/cm². Further, the second drug layered pellets is dried in the fluid bed coater for 30 minutes at predetermined parameters. As used herein, predetermined parameters include an inlet temperature of about 40°C to about 60°C, product temperature of about 30°C to about 40°C. Next, the dried first drug layered pellets are sifted/sieved in the vibratory sifter of about ASTM 14 to about ASTM 30 to form the dried and sieved second drug layered pellets. Theoretical weight of the second drug layered pellets is 3235.25g while practical weight of the second drug layered pellets is 3148.5g giving the yield of 97.3%.

The second drug layered pellets formed in the process described above have an appearance of light to dark beige spherical pellets. Theoretical assay of the second drug layered pellets is 683.8 mg/g while the practical assay of the second drug layered pellets is 670.1 mg/g having % yield of 98.0.

In TABLE 3E below, the different ingredients to prepare second drug layering suspension and performing second drug layering are depicted. Further, TABLE 3F below shows the analytical results of the second drug layered pellets.

**TABLE 3E**

| Ingredients | Qty. in (g) |
|---|---|
| Seal coated pellets | 650 |
| Mesalamine | 1964.75 |
| HPMC 603 | 589.42 |
| Simethicone 30% | 38.63 |
| | (Solid 11.59) |
| Tween / Polysorbate 80 | 19.49 |
| Purified water* | 7525.0 |
| | |
| Theoretical weight | 3235.25 |
| Practical weight | 3148.5 |
| % Yield | 97.3 |

**TABLE 3F**

| Appearance | Light to dark beige spherical pellets |
|---|---|
| Theoretical assay | 683.8 mg/g |
| Practical assay | 670.1 mg/g |
| % | 98.0 % |

Next, extended release coating suspension is formed by dissolving 9.46g of ethocel N 20 (ethyl cellulose N20) in to 245.63g of isopropyl alcohol under stirring/ homogenizing conditions in a propeller stirrer/homogenizer from medium to high speed for about 30 minutes or till cloudy suspension is formed. Thereafter, 6.31g of povidone K 30(PVP K-30) is added in to above ethocel N 20 solution under stirring/ homogenizing conditions in a propeller stirrer/homogenizer from medium to high speed for about 15 minutes or till clear to cloudy suspension is formed. Further, 81.09g of purified water is added in to above solution under stirring/ homogenizing conditions in a propeller stirrer/homogenizer from medium to high speed for about 10 minutes or till clear to cloudy suspension is formed. Next, 1.75g of dibutyl sebacate and 3.50g of purified talc is added in to above solution under stirring/ homogenizing conditions in a propeller stirrer/homogenizer from medium to high speed for about 15 minutes or till clear to cloudy suspension is formed. This cloudy suspension formed is the extended release coating suspension.

Next, extended release coating is performed by applying the extended release coating suspension on 350g of second drug layered pellets in fluid bed coater at predetermined parameters to form extended release pellets. As used herein, predetermined parameters include an inlet temperature of about 30°C to about 50°C, product temperature of about 25°C to about 35°C, fluidization of air volume of about 600 CFM to about 950 CFM, spraying rate of the seal coating suspension of about 0.5 grams to about 2 grams per minute and atmospheric pressure of about 1.0 kg/cm² to about 2.0 kg/cm². Further, the extended release pellets is dried in the fluid bed coater for 30 minutes at predetermined parameters. As used herein, predetermined parameters include an inlet temperature of about 40°C to about 50°C, product temperature of about 30°C to about 40°C. Next, the dried extended release pellets are sifted/sieved in the vibratory sifter of about ASTM 14 to about ASTM 30 to form the dried and sieved extended release pellets. The total % of polymer applied to control the release pattern of the second drug layered pellets is 6%. Theoretical weight of the extended release pellets is 371.00g while practical weight of the extended release pellets is 368.00g giving the yield of 99.21%.

The extended release pellets in the process described above have an appearance of light to dark beige spherical pellets. The mean assay (practical) of the extended release pellets is 651.68mg/g.

In TABLE 3G below, the different ingredients to prepare extended release coating suspension and performing extended release coating are depicted. Further, TABLE 3H below shows the analytical results of the extended release pellets.

**TABLE 3G**

| Ingredients | Qty. in (g) |
|---|---|
| Total % of polymer applied | 6% |
| second drug layered pellets | 350 |
| Ethyl cellulose N20 | 9.46 |
| PVP K-30 | 6.31 |
| Dibutyl sebaccate | 1.75 |
| Talcum | 3.50 |
| Isopropyl alcohol | 245.63 |
| Purified water | 81.09 |
| | |
| Theoretical weight | 371.00 |
| Practical weight | 368.00 |
| % Yield | 99.21 % |

**TABLE 3H**

| Appearance | | Light to dark beige spherical pellets |
|---|---|---|
| Mean assay (practical) | | 651.68 mg/gm |
| Dissolution Paddle 100 RPM. 900 ml of Phosphate buffer pH 7.5 | 1 Hr | 13.1 |
| | (NMT 30%) | |
| | 2 Hr | 30.1 |
| | (20% - 60 %) | |
| | 4 Hr | 60.9 |
| | (40% - 80 %) | |
| | 8 Hr | 89.9 |
| | (NLT 75%) | |

Next, the color coating suspension is formed by suspending 16.25g of colorcon opadry yellow color in 146.25g of purified water under stirring/ homogenizing conditions in a propeller stirrer/homogenizer from medium to high speed for about 30 minutes or till yellow color cloudy suspension is formed. This yellow color cloudy suspension formed is the color coating suspension.

Thereafter, color coating is performed by applying the color coating suspension on 325g of extended release pellets in fluid bed coater at predetermined parameters to form color coated pellets. As used herein, predetermined parameters include an inlet temperature of about 30°C to about 50°C, product temperature of about 25°C to about 35°C, fluidization of air volume of about 600 CFM to about 950 CFM, spraying rate of the first drug layering suspension of about 0.5 grams to about 2 grams per minute and atmospheric pressure of about 1.0 kg/cm² to about 2.0 kg/cm². Further, the color coated pellets formed are dried in the fluid bed coater for 30 minutes at predetermined parameters. As used herein, predetermined parameters include an inlet temperature of about 40°C to about 50°C, product temperature of about 30°C to about 40°C. Next, the dried color coated pellets are sifted/sieved in the vibratory sifter of about ASTM 14 to about ASTM 30 to form the dried and sieved color coated pellets. Theoretical weight of the color coated pellets is 341.25g while practical weight of the color coated pellets is 333.00g giving the yield of 97.82%.

The color coated pellets in the process described above have an appearance of yellow to yellowish spherical pellets. The mean assay (practical) of the color coated pellets is 605.0mg/g.

In TABLE 31 below, the different ingredients to prepare color coating suspension and performing color coating are depicted. Further, TABLE 3J below shows the analytical results of the color coated pellets.

**TABLE 3I**

| Ingredients | Qty. in (g) |
|---|---|
| Extended release pellets | 325 |
| Instacoat / Colorcon coloring material | 16.25 |
| Purified water | 146.25 |
| | |
| Theoretical weight | 341.25 |
| Practical weight | 333.00 |
| % Yield | 97.82 % |

**TABLE 3J**

| Appearance | | Yellow to yellowish spherical pellets |
|---|---|---|
| Mean assay (practical) | | 605.0 mg/gm |
| Dissolution Paddle 100 RPM. 900 ml of Phosphate buffer pH 7.5 | 1 Hr | 14.2 |
| | (NMT 30%) | |
| | 2 Hr | 34.1 |
| | (20% - 60 %) | |
| | 4 Hr | 64.6 |
| | (40% - 80 %) | |
| | 8 Hr | 95.5 |
| | (NLT 75%) | |

Thereafter, the color coated pellets formed above is lubricated with about 0.5% talcum in coating pan at slow speed of 8rpm for 3 minutes to form mesalamine pellets.

The percentage of the final lubricated mesalamine pellets formed in the above examples passing through the sieve number of ASTM 14 is not less than 95%. Further , The percentage of the final lubricated mesalamine pellets formed in the above examples retained through the sieve numbers of ASTM 30 is not less than 95% .Further, the particle size distribution of the final lubricated mesalamine pellets formed in the above examples were found of uniform particle size. Accordingly, the present examples provide a faster, easier, robust, automated and reproducible process for preparation of a mesalamine composition.

In TABLE 4 below, the particle size distribution of the final lubricated mesalamine pellets formed in the above examples is depicted. A uniform particle size distribution of all batches were found that ensure a reproducible and riobust process.

**TABLE 4**

| Examples | Example 1 | Example 2 | Example 3 |
|---|---|---|---|
| Sieve numbers | Particle size distribution | | |
| NLT 95% particle passes through 14# | Complies | Complies | Complies |
| NLT 95% particle retained on 30# | Complies | Complies | Complies |

Further, the bulk/tapped density of the final lubricated mesalamine pellets formed in the above examples are found consistent. In TABLE 5 below, the bulk density of the final lubricated mesalamine pellets formed in the above examples is depicted. As per the observation in TABLE 5, the bulk/tapped density is found consistent for all batches.

**TABLE 5**

| Examples | Example 1 | Example 2 | Example 3 |
|---|---|---|---|
| Bulk density | 0.85 | 0.82 | 0.85 |

All the process for manufacturing mesalamine pellets as described in the above examples were done in the fluid bed coater; the practical yield of the mesalamine pellets formed were observed more than 95%.

## Claims

1. A process for preparation of a mesalamine composition, wherein the mesalamine composition comprises
about 55 to about 65 percent by weight of a mesalamine;
about 7 to about 9 percent by weight of a sugar spheres ;
about 17 to about 19 percent by weight of a binder, wherein the binder is hypromellose;
about 0.50 to about 0.80 percent by weight of a surfactant, wherein the surfactant is polysorbate 80;
about 1 to about 1.5 percent by weight of an anti foaming agent;
about 4 to about 5 percent by weight of a release controlling agent, wherein the release controlling agent is ethyl cellulose;
about 2 to about 3 percent by weight of a pore forming agent, wherein the pore forming agent is polyvinyl pyrrolidone.;
about 0.5 to about 1 percent by weight of a plasticizer, wherein the plasticizer is dibutyl sebacate;
about 1.5 to about 3 percent by weight of a lubricant, wherein the lubricant is purified talc; and
about 2.5 to about 3 percent by weight of a colorant
comprising:
preparing a first drug layering suspension comprising mesalamine;
performing a first drug layering in a fluid bed coater by applying the first drug layering suspension on sugar spheres to form first drug layered pellets;
preparing a seal coating suspension;
performing a seal coating in the fluid bed coater by applying the seal coating suspension on the first drug layered pellets to form seal coated pellets;
preparing a second drug layering suspension comprising mesalamine;
performing a second drug layering in the fluid bed coater by applying the second drug layering suspension on seal coated pellets to form second drug layered pellets;
preparing an extended release coating suspension;
performing an extended release coating in the fluid bed coater by applying extended release coating suspension on the second drug layered pellets to form extended release pellets;
preparing a color coating suspension;
performing a color coating in the fluid bed coater by applying the color coating suspension on the extended release pellets to form color coated pellets; and
lubricating the color coated pellets.

2. The process of claim 1, wherein the preparation of first drug layering suspension comprises mixing binder, water, anti foaming agent, surfactant and mesalamine.

3. The process of claim 1, wherein the preparation of second drug layering suspension comprises mixing binder, water, anti foaming agent, surfactant and mesalamine.

4. The process of claim 1, wherein the preparation of seal coating suspension comprises mixing release controlling agent, isopropyl alcohol, pore forming agent, water, plasticizer and lubricant.

5. The process of claim 1, wherein the preparation of extended release coating suspension comprises mixing release controlling agent, isopropyl alcohol, pore forming agent, water, plasticizer and lubricant.

6. The process of claim 1, wherein the preparation of second color coating suspension comprises mixing colorant and water.

7. The process of claims 1, 2 or 3, wherein the anti foaming agent is simethicone 30% emulsion.

8. The process of claim 1, wherein the sugar spheres is of the size about 300µm to about 500µm.

9. A mesalamine composition, comprising:
about 55 to about 65 percent by weight of a mesalamine;
about 7 to about 9 percent by weight of sugar spheres;
about 17 to about 19 percent by weight of a binder, wherein the binder is hypromellose;
about 0.50 to about 0.80 percent by weight of a surfactant, wherein the surfactant is polysorbate 80;
about 1 to about 1.5 percent by weight of an anti foaming agent;
about 4 to about 5 percent by weight of a release controlling agent, wherein the release controlling agent ethyl cellulose;
about 2 to about 3 percent by weight of a pore forming agent, wherein the pore forming agent is polyvinyl pyrrolidone;
about 0.5 to about 1 percent by weight of a plasticizer, wherein the plasticizer is dibutyl sebacate;
about 1.5 to about 3 percent by weight of a lubricant, wherein the lubricant is purified talc; and
about 2.5 to about 3 percent by weight of a colorant.

## Patentansprüche

1. Verfahren zur Herstellung einer Mesalamin-Zusammensetzung, wobei die Mesalamin-Zusammensetzung umfasst:
etwa 55 bis etwa 65 Gewichtsprozent eines Mesalamins;
etwa 7 bis etwa 9 Gewichtsprozent Zuckerkügelchen;
etwa 17 bis etwa 19 Gewichtsprozent eines Bindemittels, wobei das Bindemittel Hypromellose ist;
etwa 0,50 bis etwa 0,80 Gewichtsprozent eines Tensids, wobei das Tensid Polysorbat 80 ist;
etwa 1 bis etwa 1,5 Gewichtsprozent eines Antischaummittels;
etwa 4 bis etwa 5 Gewichtsprozent eines Freisetzungskontrollmittels, wobei das Freisetzungskontrollmittel Ethylcellulose ist;
etwa 2 bis etwa 3 Gewichtsprozent eines Porenbildners, wobei der Porenbildner Polyvinylpyrrolidon ist;
etwa 0,5 bis etwa 1 Gewichtsprozent eines Weichmachers, wobei der Weichmacher Dibutylsebacat ist;
etwa 1,5 bis etwa 3 Gewichtsprozent eines Schmiermittels, wobei das Schmiermittel gereinigtes Talkum ist; und
etwa 2,5 bis etwa 3 Gewichtsprozent eines Farbstoffs,
umfassend:
Herstellen einer ersten Arzneimittel-Beschichtungssuspension, die Me-salamin enthält;
Durchführen einer ersten Arzneimittelbeschichtung in einem Fließbettbeschichter durch Auftragen der ersten Arzneimittel-Beschichtungssuspension auf Zuckerkügelchen, um erste arzneimittelgeschichtete Pellets zu bilden;
Herstellen einer Versiegelungsbeschichtungssuspension;
Durchführen einer Versiegelungsbeschichtung im Fließbettbeschichter durch Auftragen der Versiegelungsbeschichtungssuspension auf die ersten Arzneimittel-Schichtpellets, um versiegelungsbeschichtete Pellets zu bilden;
Herstellen einer zweiten Arzneimittelschichtungssuspension, die Mesalamin enthält;
Durchführen einer zweiten Arzneimittelschichtung im Fließbettbeschichter durch Auftragen der zweiten Arzneimittel-Beschichtungssuspension auf versiegelungsbeschichtete Pellets, um zweite arzneimittelbeschichtete Pellets zu bilden;
Herstellen einer Beschichtungssuspension mit verlängerter Freisetzung;
Durchführen einer Beschichtung mit verlängerter Freisetzung im Fließbettbeschichter durch Auftragen einer Beschichtungssuspension mit verlängerter Freisetzung auf die zweiten arzneimittelbeschichteten Pellets, um Pellets mit verlängerter Freisetzung zu bilden;
Herstellen einer Farbbeschichtungssuspension;
Durchführen einer Farbbeschichtung im Fließbettbeschichter durch Auftragen der Farbbeschichtungssuspension auf die Pellets mit verlängerter Freisetzung, um farbbeschichtete Pellets zu bilden; und Schmierung der farbbeschichteten Pellets.

2. Verfahren nach Anspruch 1, wobei die Herstellung der ersten Arzneimittelschichtungssuspension Mischen von Bindemittel, Wasser, Antischaummittel, Tensid und Mesalamin umfasst.

3. Verfahren nach Anspruch 1, wobei die Herstellung einer zweiten Arzneimittelschichtungssuspension Mischen von Bindemittel, Wasser, Antischaummittel, Tensid und Mesalamin umfasst.

4. Verfahren nach Anspruch 1, wobei die Herstellung einer Siegelbeschichtungssuspension Mischen von Freisetzungskontrollmittel, Isopropylalkohol, Porenbildner, Wasser, Weichmacher und Schmiermittel umfasst.

5. Verfahren nach Anspruch 1, wobei die Herstellung einer Beschichtungssuspension mit verlängerter Freigabe Mischen von Freisetzungskontrollmittel, Isopropylalkohol, Porenbildner, Wasser, Weichmacher und Schmiermittel umfasst.

6. Verfahren nach Anspruch 1, wobei die Herstellung einer zweiten Farbbeschichtungssuspension Mischen von Farbstoff und Wasser umfasst.

7. Verfahren nach den Ansprüchen 1, 2 oder 3, wobei das Antischaummittel eine 30%ige Simethicon-Emulsion ist.

8. Verfahren nach Anspruch 1, wobei die Zuckerkügelchen von einer Größe von etwa 300µm bis etwa 500µm sind.

9. Mesalamin-Zusammensetzung, umfassend:
etwa 55 bis etwa 65 Gewichtsprozent eines Mesalamins;
etwa 7 bis etwa 9 Gewichtsprozent Zuckerkügelchen;
etwa 17 bis etwa 19 Gewichtsprozent eines Bindemittels, wobei das Bindemittel Hypromellose ist;
etwa 0,50 bis etwa 0,80 Gewichtsprozent eines Tensids, wobei das Tensid Polysorbat 80 ist;
etwa 1 bis etwa 1,5 Gewichtsprozent eines Antischaummittels;
etwa 4 bis etwa 5 Gewichtsprozent eines Freisetzungskontrollmittels, wobei das Freisetzungskontrollmittel Ethylcellulose ist;
etwa 2 bis etwa 3 Gewichtsprozent eines Porenbildners, wobei der Porenbildner Polyvinylpyrrolidon ist;
etwa 0,5 bis etwa 1 Gewichtsprozent eines Weichmachers, wobei der Weichmacher Dibutylsebacat ist;
etwa 1,5 bis etwa 3 Gewichtsprozent eines Schmiermittels, wobei das Schmiermittel gereinigtes Talkum ist; und
etwa 2,5 bis etwa 3 Gewichtsprozent eines Farbstoffs.

## Revendications

1. Procédé de préparation d'une composition de mésalamine, dans lequel la composition de mésalamine comprend
environ 55 à environ 65 pour cent en poids de mésalamine ;
environ 7 à environ 9 pour cent en poids de sphères de sucre ;
environ 17 à environ 19 pour cent en poids d'un liant, où le liant est l'hypromellose ;
environ 0,50 à environ 0,80 pour cent en poids d'un tensioactif, où le tensioactif est le polysorbate 80 ;
environ 1 à environ 1,5 pour cent en poids d'un agent anti-mousse ;
environ 4 à environ 5 pour cent en poids d'un agent de régulation de libération, où l'agent de régulation de libération est l'éthylcellulose ;
environ 2 à environ 3 pour cent en poids d'un agent porogène, où l'agent porogène est la polyvinylpyrrolidone ;
environ 0,5 à environ 1 pour cent en poids d'un plastifiant, où le plastifiant est le sébacate de dibutyle ;
environ 1,5 à environ 3 pour cent en poids d'un lubrifiant, où le lubrifiant est du talc purifié ; et
environ 2,5 à environ 3 pour cent en poids d'un colorant
comprenant le fait :
de préparer une première suspension de médicament à stratifier comprenant de la mésalamine ;
d'effectuer une première stratification de médicament dans un dispositif d'enrobage à lit fluidisé en appliquant la première suspension de médicament à stratifier sur les sphères de sucre pour former des premières pastilles de médicament stratifiées ;
de préparer une suspension d'enrobage étanche ;
d'effectuer un enrobage étanche dans le dispositif d'enrobage à lit fluidisé en appliquant la suspension d'enrobage étanche sur les premières pastilles de médicament stratifiées pour former des pastilles à enrobage étanche ;
de préparer une deuxième suspension de médicament à stratifier comprenant de la mésalamine ;
d'effectuer une deuxième stratification de médicament dans le dispositif d'enrobage à lit fluidisé en appliquant la deuxième suspension de médicament à stratifier sur des pastilles à enrobage étanche pour former des deuxièmes pastilles de médicament stratifiées ;
de préparer une suspension d'enrobage à libération prolongée ;
d'effectuer un enrobage à libération prolongée dans le dispositif d'enrobage à lit fluidisé en appliquant une suspension d'enrobage à libération prolongée sur les deuxièmes pastilles de médicament stratifiées pour former des pastilles à libération prolongée ;
de préparer une suspension d'enrobage coloré ;
d'effectuer un enrobage coloré dans le dispositif d'enrobage à lit fluidisé en appliquant la suspension d'enrobage coloré sur les pastilles à libération prolongée pour former des pastilles à enrobage coloré ; et
de lubrifier les pastilles à enrobage coloré.

2. Procédé de la revendication 1, dans lequel la préparation de la première suspension de médicament à stratifier comprend le mélange de liant, d'eau, d'agent anti-mousse, de tensioactif et de mésalamine.

3. Procédé de la revendication 1, dans lequel la préparation de la deuxième suspension de médicament à stratifier comprend le mélange de liant, d'eau, d'agent anti-mousse, de tensioactif et de mésalamine.

4. Procédé de la revendication 1, dans lequel la préparation d'une suspension d'enrobage étanche comprend le mélange d'agent de régulation de libération, d'alcool isopropylique, d'agent porogène, d'eau, de plastifiant et de lubrifiant.

5. Procédé de la revendication 1, dans lequel la préparation d'une suspension d'enrobage à libération prolongée comprend le mélange d'agent de régulation de libération, d'alcool isopropylique, d'agent porogène, d'eau, de plastifiant et de lubrifiant.

6. Procédé de la revendication 1, dans lequel la préparation de la deuxième suspension d'enrobage coloré comprend le mélange de colorant et d'eau.

7. Procédé de la revendication 1, 2 ou 3, dans lequel l'agent anti-mousse est une émulsion de siméthicone 30%.

8. Procédé de la revendication 1, dans lequel les sphères de sucre ont une taille allant d'environ 300 µm à environ 500 µm.

9. Composition de mésalamine, comprenant :
environ 55 à environ 65 pour cent en poids de mésalamine ;
environ 7 à environ 9 pour cent en poids de sphères de sucre ;
environ 17 à environ 19 pour cent en poids d'un liant, où le liant est l'hypromellose ;
environ 0,50 à environ 0,80 pour cent en poids d'un tensioactif, où le tensioactif est le polysorbate 80 ;
environ 1 à environ 1,5 pour cent en poids d'un agent anti-mousse ;
environ 4 à environ 5 pour cent en poids d'un agent de régulation de libération, où l'agent de régulation de libération est l'éthylcellulose ;
environ 2 à environ 3 pour cent en poids d'un agent porogène, où l'agent porogène est la polyvinylpyrrolidone ;
environ 0,5 à environ 1 pour cent en poids d'un plastifiant, où le plastifiant est le sébacate de dibutyle ;
environ 1,5 à environ 3 pour cent en poids d'un lubrifiant, où le lubrifiant est du talc purifié ; et
environ 2,5 à environ 3 pour cent en poids d'un colorant.
